(19) Europäisches Patentamt
European Patent Office
Office européen des brevets

(11) Veröffentlichungsnummer: **0 112 798**
**B1**

(12) # EUROPÄISCHE PATENTSCHRIFT

(45) Veröffentlichungstag der Patentschrift: **13.05.87**

(21) Anmeldenummer: **83810543.5**

(22) Anmeldetag: **21.11.83**

(51) Int. Cl.⁴: **G 03 C 5/00,** C 08 G 85/00, C 09 B 1/54, C 09 B 69/00, C 09 B 62/54, C 08 G 59/62, C 07 C 97/24, C 07 D 303/32

(54) **Zu Kondensations- oder Additionsreaktionen befähigte lichtempfindliche und gegebenenfalls vernetzbare Stoffgemische, daraus herstellbare Reaktionsprodukte und deren Verwendung.**

(30) Priorität: **25.11.82 CH 6870/82**

(43) Veröffentlichungstag der Anmeldung: **04.07.84 Patentblatt 84/27**

(45) Bekanntmachung des Hinweises auf die Patenterteilung: **13.05.87 Patentblatt 87/20**

(84) Benannte Vertragsstaaten: **CH DE FR GB LI NL**

(56) Entgegenhaltungen: **GB - A - 1 119 140**

(73) Patentinhaber: **CIBA-GEIGY AG, Klybeckstrasse 141, CH-4002 Basel (CH)**

(72) Erfinder: **Finter, Jürgen, Dr., Zasiusstrasse 100, D-7800 Freiburg (DE)**
Erfinder: **Fischer, Walter, Dr., Vogesenstrasse 77, CH-4153 Reinach (CH)**
Erfinder: **Lohse, Friedrich, Prof. Dr., Buchenstrasse 23, CH-4104 Oberwil (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft neue zu Kondensations- oder Additionsreaktionen befähigte lichtempfindliche und gegebenenfalls vernetzbare Stoffgemische, daraus herstellbare Reaktionsprodukte und Verfahren zu deren Herstellung sowie die Verwendung der neuen lichtempfindlichen Stoffgemische und Reaktionsprodukte, vor allem zur Bilderzeugung.

Elektrisch leitende Überzüge und Muster, vor allem für gedruckte Schaltungen, können unter anderem dadurch hergestellt werden, dass man auf nicht-leitenden anorganischen oder organischen Substraten für die stromlose Metallabscheidung geeignete nullwertige Metallkeime erzeugt. Nach dem sogenannten Photoformation-Verfahren kann dies dadurch erfolgen, dass man auf dem Substrat, gegebenenfalls in saurem Medium und in Gegenwart von Halogenidionen, Metallsalze, besonders Salze nicht-edler Metalle, wie Kupferformiat, abscheidet und anschliessend durch Bestrahlung, gegebenenfalls in Gegenwart chemischer Reduktionsmittel, zu nullwertigen Metallkeimen reduziert. Für die Reduktion der Metallsalze werden im allgemeinen lichtempfindliche Reduktionsmittel, ein zweites Reduktionsmittel und ein oberflächenaktives Mittel eingesetzt. Als lichtempfindliche Reduktionsmittel können u.a. Anthrachinondisulfonsäuren und Salze davon, gegebenenfalls im Gemisch mit Metallaktivatoren, wie Zinnsalzen, eingesetzt werden. Dabei müssen die Substrate im allgemeinen vor der Metallabscheidung angeätzt oder mit einer ätzbaren Haftvermittlerschicht versehen werden [intramolekulare photoreduktive Methode; vgl. z.B. U.S. Patentschriften 3 959 547 und 3 993 802].

Gemäss einem weiteren vorbekannten Verfahren wird auf dem nichtleitenden Substrat eine Titandioxid enthaltende lichtempfindliche Schicht erzeugt oder Titandioxid wird in das Substrat eingearbeitet. Anschliessend muss das das Titandioxid enthaltende Substrat bzw. die das Titandioxid enthaltende Schicht angeätzt werden, um die $TiO_2$-Teilchen für die weitere Behandlung zugänglich zu machen. Das angeätzte Material wird dann mit einer Lösung des gewünschten Metallsalzes behandelt und bestrahlt (Photoelektronen-Methode). Schliesslich können nullwertige Metallkeime auch dadurch erhalten werden, dass man auf dem nicht-leitenden Substrat zuerst ein lichtempfindliches Metallsalz, wie $SnCl_2$ oder Eisenoxalat, abscheidet, durch Bestrahlung ein latentes Bild bzw. reduzierende Metallionen erzeugt und darauf durch Reduktion eines Metallsalzes, im allgemeinen eines Edelmetallsalzes, die nullwertigen Metallkeime erzeugt (photoelektrochemische Methode). Die so erhaltenen nullwertigen Metallkeime können anschliessend in an sich bekannter Weise durch stromlose Metallabscheidung metallisiert und die leitenden Bildstellen gegebenenfalls durch elektrolytische Metallabscheidung weiter verstärkt werden.

In der GB-A-1 119 140 sind Reaktionsprodukte aus einem Epoxidharz und einem hydroxysubstituierten Chinon beschrieben. Als Chinon wird zum Beispiel Hydroxyanthrachinon erwähnt. Die Reaktionsprodukte dienen als photoleitfähiges Material zur Aufzeichnung von Informationen, beispielsweise in der Xerographie.

Es wurden nun neue Anthrachinone enthaltende lichtempfindliche Stoffgemische gefunden, mit denen bzw. den daraus herstellbaren Reaktionsprodukten Abbildungen, besonders elektrisch leitende Überzüge und Muster, auf wesentlich einfachere und wirtschaftlichere Weise hergestellt werden können, wobei auf das Anätzen des Substrates oder die Verwendung von ätzbaren Haftvermittlerschichten verzichtet werden kann. Mit den neuen Stoffgemischen bzw. Reaktionsprodukten erzeugte Abbildungen zeichnen sich zudem durch ein erhöhtes Auslösungsvermögen aus.

Gegenstand der Erfindung sind somit neue zur Kondensations- oder Additionsreaktionen befähigte lichtempfindliche und gegebenenfalls verneztbare Stoffgemische, enthaltend

1) ein Anthrachinon der Formel I

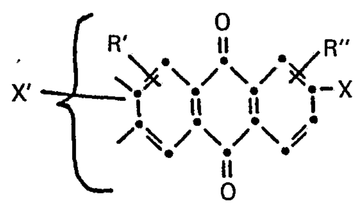

(I),

2) eine oder mehrere Verbindungen ausgewählt aus Di- bis Polyglycidyläthern von Phenol- und Kresolnovolacken und Verbindungen der Formeln II bis VIII

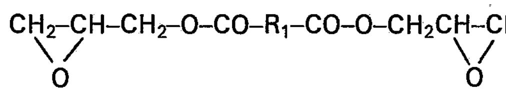

(II),

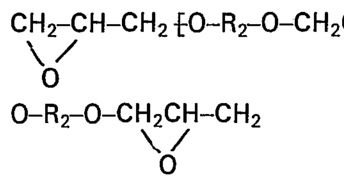

III),

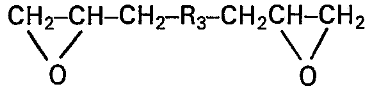

(IV),

$$HO{-}R_2{-}O\,[CH_2CH(OH)CH_2{-}O{-}R_2{-}O]_{b'}{-}H \quad (V),$$

$$HO{-}R{-}O\,[CO{-}R_1{-}CO{-}O{-}R{-}O]_a{-}H \quad (VI),$$

$$HOOC{-}R_1{-}CO\,[O{-}R{-}O{-}CO{-}R_1{-}CO]_aOH \quad (VII)$$

und

$$HO\,[Y_3{-}O]_o{-}Y_3{-}OH \quad (VIII),$$

wobei der Anteil an Verbindungen der Formeln VI, VII und/oder VIII höchstens 80 Mol%, bezogen auf alle unter 2) genannten Reaktionskomponenten beträgt,

3) gegebenenfalls ein Vernetzungsmittel und

4) gegebenenfalls ein Salz eines Metalls der Gruppe Ib oder VIII des periodischen Systems, worin

R' und R'' unabhängig voneinander Wasserstoff, Methyl, Halogen, eine Nitro-, Phenylsulfonyl- oder Methoxygruppe bedeuten,

X $-COOH$, $-COCl$, $-O-C_pH_{2p}-COOH$ mit $p = 1$ bis 4, $-NH_2$, $-OH$, $-CH_2NH_2$, $-CH_2OH$ oder $-OCH_2CH-CH_2$ darstellt,

X' Wasserstoff bedeutet oder dieselbe Bedeutung wie X hat, mit Ausnahme von Hydroxyanthrachinon,

a eine Zahl von 1–100, besonders 2–50,

b eine Zahl von 0–150, besonders 0,1–150 und vor allem 2–100,

b' eine Zahl von 0,1–150, besonders 2–100,

R $-C_mH_{2m}-$ mit $m = 2–12$, $-[CH_2CH_2O]_r-CH_2CH_2-$ mit $r = 1–40$, besonders 1–20, $-CH(CH_3)CH_2OCH_2CH(CH_3)-$, Cyclohexylen, $-CH_2-C(CH_3)_2-OCOC(CH_3)_2CH_2-$,

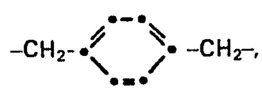 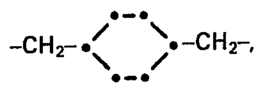

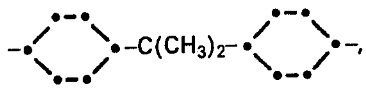 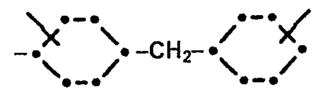 ,

Naphthylen, Biphenylen oder gegebenenfalls durch eine Methyl-, Methoxy- oder Nitrogruppe substituiertes Phenylen,

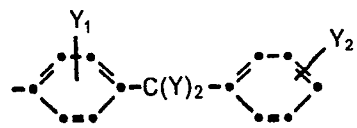 oder $-(Y_3O)_o-Y_3-$ ,

$R_1$ die direkte Bindung, $-C_mH_{2m}-$ mit $m = 2–12$ oder Cyclohexylen, Cyclohexenylen, Phenylen oder Endomethylencyclohexenylen, die durch eine Methylgruppe substituiert sein können,

$R_2$ $-C_mH_{2m}-$ mit $m = 2–12$, Phenylen,

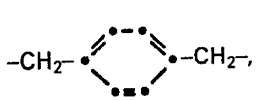 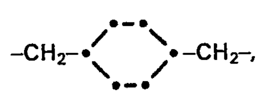

eine Gruppe der Formeln

Y Wasserstoff oder Methyl,

$Y_1$ und $Y_2$ unabhängig voneinander Wasserstoff, Chlor oder Brom,

$Y_3$ $-(CH_2)_2-$, $-CH_2CH(CH_3)-$ oder $-(CH_2)_4-$,

o eine Zahl von 1–50 und

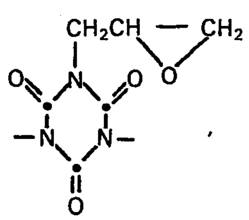 , 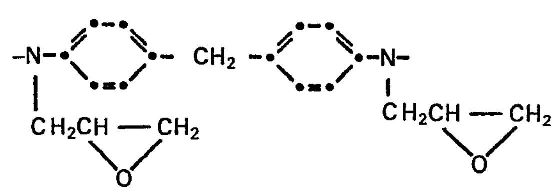

den Rest des Äthylenharnstoffs, 1,3-Propylenharnstoffs, 5,5-Dimethylhydantoins, 2-Hydroxyäthyl-5,5-dimethylhydantoins oder 2-Hydroxypropyl-5,5-dimethylhydantoins bedeuten.

Die erfindungsgemässen Stoffgemische müssen definitionsgemäss zu Kondensations- oder Additionsreaktionen befähigt sein. Daher stellen X bzw. X' bei Verbindungen der Formeln V, VI und/oder VIII z.B. $-COCl$, $-O-C_pH_{2p}-COOH$ oder $-OCH_2CH-CH_2$, bei Verbindungen mit Glycidylendgruppen $-OH$, $-NH_2$, $-CH_2NH_2$, $-CH_2OH$, $-COOH$ oder $-O-C_pH_{2p}-COOH$ und bei Verbindungen der Formel VII z.B. $-OCH_2CH-CH_2$, $-NH_2$, $-OH$, $-CH_2-H_2$ oder $-CH_2OH$ dar.

Der Anteil an Verbindungen der Formel I beträgt zweckmässig zwischen 1 und 60 Mol%, vorzugsweise 2 bis 45 Mol%, bezogen auf die unter 2) angegebenen Verbindungen.

Sind a, b oder b' grösser als 1, so können die einzelnen R, $R_1$, $R_2$ und $Y_3$ in den Verbindungen der Formel III und V bis VIII (Oligomere bzw. Polymere) gleiche oder unterschiedliche Bedeutungen haben, und wiederkehrende Strukturelemente in solchen Oligomeren oder Polymeren können statistisch oder blockartig angeordnet sein.

Stellen R' und/oder R'' Halogen dar, so handelt es sich insbesondere um Chlor- oder Bromatome. Besonders bevorzugt stellen R und R' je Wasserstoff dar.

Durch X, X', R, $R_1$ oder $R_2$ dargestellte Gruppen

$-C_pH_{2p}-$ oder $-C_mH_{2m}-$ können geradkettig oder verzweigt sein;

p ist vorzugsweise 1 oder 2 und m bevorzugt 2–10.

Als Beispiele solcher Gruppen seien genannt: $-CH_2-$, $-(CH_2)_2-$, $-CH_2CH(CH_3)-$, $-(CH_2)_3-$, $-(CH_2)_4-$, $-(CH_2)_2-C(CH_3)_2-(CH_2)_2-$, $-(CH_2)_6-$, $-C(CH_3)_2-$, $-CH_2C(CH_3)_2-CH_2-CH(CH_3)(CH_2)_2-$, $-CH_2CH(CH_3)CH(CH_3)CH_2CH(CH_3)CH_2-$, $-(CH_2)_7-$, $-(CH_2)_8-$, $-(CH_2)_{10}-$ und $-(CH_2)_{12}-$.

Stellen R und/oder $R_2$ Gruppen $-C_mH_{2m}-$ dar, so handelt es sich insbesondere um Reste des Äthylenglykols, 1,4-Butandiols, Neopentylglykols oder 1,6-Hexandiols. Ist $R_1$ eine Gruppe $-C_mH_{2m}-$, so kommen vor allem Reste der Bernsteinsäure-, Adipinsäure, Pimelinsäure, Azelainsäure oder Sebacinsäure in Betracht.

Bedeutet R Cyclohexylen, so handelt es sich insbesondere um den Rest des 1,2-Cyclohexandiols. Als Cyclohexylengruppen $R_1$ kommen vor allem 1,3- und insbesondere 1,4-Cyclohexylen in Betracht, die durch Methyl substituiert sein können, bevorzugt aber unsubstituiert sind.

Stellt R Naphthylen, Biphenylen oder gegebenenfalls durch eine Methyl-, Methoxy- oder Nitrogruppe substituiertes Phenylen dar, so kommen z.B. Reste des 1,4-, 1,6-, 1,8- und 2,6-Dihydroxynaphthalins, des 2,2'-Biphenyls, Resorcins, 2,5-Dihydroxyanisols, 1,2-Dihydroxy-4-nitrobenzols, 2,5- und 3,4-Dihydroxytoluols in Betracht. Bevorzugt bedeutet R $-(CH_2)_2-$, $-(CH_2)_4-$, $-(CH_2)_6-$, $-CH_2CH_2OCH_2CH_2-$, $-C(CH_3)_2-$,

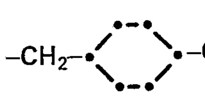 oder 1,3-Phenylen.

Bedeutet $R_1$ gegebenenfalls durch Methyl substituiertes Cyclohexenylen, Phenylen oder Endomethylencyclohexenylen, so handelt es sich beispielsweise um Reste der Methyl-tetrahydrophthalsäure, Endomethylentetrahydrophthalsäure, Tetrahydrophthalsäure, Phthalsäure, Isophthalsäure oder Terephthalsäure. $R_1$ ist bevorzugt $-(CH_2)_m$ mit m = 2–10, 1,3- oder 1,4-Phenylen, 1,3- oder 1,4-Cyclohexylen.

Ist $R_2$ Phenylen, so handelt es sich insbesondere um 1,3-Phenylen.

Bedeutet $R_2$ eine Gruppe der Formel

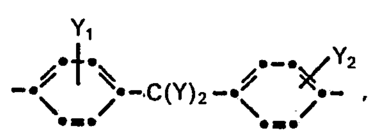 ,

so stellen $Y_1$ und $Y_2$ vorzugsweise je in 2,2'-Stellung gebundenes Chlor oder Brom dar. Besonders bevorzugt sind jedoch solche Gruppen, worin $Y_1$ und $Y_2$ Wasserstoff bedeuten. Stellt $R_2$ eine Gruppe $-(Y_3O)_o-Y_3$ dar, so ist $Y_3$ bevorzugt $-(CH_2)_2-$ oder $-CH_2CH(CH_3)-$ und o stellt insbesondere 1 bis 40, vor allem 2–20, dar.

$R_2$ ist bevorzugt $-C_mH_{2m}-$ mit m = 2, 4 oder 6 oder

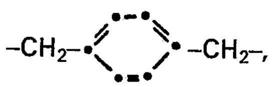 vor allem jedoch eine Gruppe der Formeln

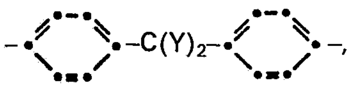 $-(CH_2CH_2O)_o-CH_2CH_2-$ und/oder $-[CH_2CH(CH_3)\ O]_o-CH_2CH(CH_3)-$ mit Y = Wasserstoff und vor allem Methyl und o = 1 bis 40, vor allem 2–20.

$R_3$ ist vorzugsweise der Rest des 5,5-Dimethylhydantoins, 2-Hydroxyäthyl- oder 2-Hydroxypropyl-5,5-dimethylhydantoins oder Triglycidylisocyanurats.

Bevorzugt sind Stoffgemische, die ein Anthrachinon der Formel I, eine oder mehrere Verbindungen mit Glycidylendgruppen und/oder eine Verbindung der Formel V, sowie gegebenenfalls ein Vernetzungsmittel und/oder definitionsgemässes Metallsalz enthalten, wobei R' und R'' Wasserstoff sind, X $-CH_2NH_2$, $-O-C_pH_{2p}-COOH$ und vor allem $-OH$ und X' Wasserstoff oder in 6-Stellung gebundenes $-O-C_pH_{2p}-COOH$ und insbesondere in 6-Stellung gebundenes $-OH$, b' 0,1 bis 100 und b eine Zahl von 1–100 bedeuten. Dabei stellt $-C_pH_{2p}-$ bevorzugt $-CH_2-$ oder $-(CH_2)_2-$ dar. Verbindungen der Formel I, worin R' und R'' Wasserstoff, X $-CH_2NH_2$ oder $-OH$ und X' Wasserstoff oder in 6-Stellung gebundenes $-OH$ bedeuten, werden vorzugsweise im Gemisch mit Verbindungen mit Glycidylendgruppen, besonders solchen der Formeln II bis IV, und gegebenenfalls Verbindungen der Formel V eingesetzt. Stellen X $-O-C_pH_{2p}-COOH$ und X' Wasserstoff oder in 6-Stellung gebundenes $-O-C_pH_{2p}-COOH$ dar, so sind Verbindungen der Formel V bevorzugt.

Besonders bevorzugt sind Gemische, die eine Verbindung der Formel I, worin R' und R'' je Wasserstoff, X $-CH_2NH_2$ und vor allem $-OH$ und X' Wasserstoff oder in 6-Stellung gebundenes $-OH$ bedeuten, eine oder mehrere Verbindungen ausgewählt aus Di- und/oder Triglycidyläthern von Phenol- oder Kresolnovolacken, Triglycidylisocyanurat, Hexahydrophthalsäurediglycidylester, N,N'-Diglycidyl-5,5-dimethylhydantoin, N-Glycidyl-N'-2-oxyäthylglycidyl- und/oder N-Glycidyl-N'-2-oxy-propylglycidyl-5,5-dimethylhydantoin und Verbindungen der Formeln A), B), C) und D)

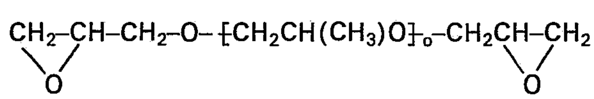

(A),

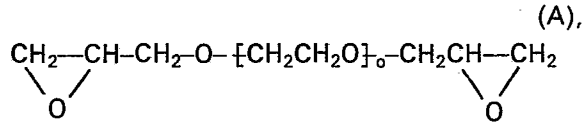

(B),

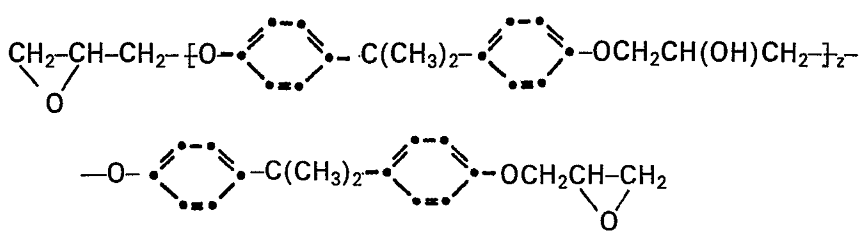

(C)

und

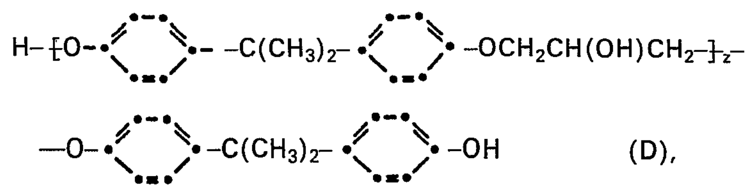

(D),

sowie gegebenenfalls ein Vernetzungsmittel und/oder ein definitionsgemässes Metallsalz enthalten, wobei o 2–40 und insbesondere 2–20 und z 0,1–13, vor allem 2–11, bedeuten.

Ganz besonders bevorzugt sind Gemische, die eine Verbindung der Formel I, worin R' und R" Wasserstoff, X –OH und X' Wasserstoff oder in 6-Stellung gebundenes –OH darstellen, eine Verbindung der Formel A) oder B) im Gemisch mit einer Verbindung der Formel C) und gegebenenfalls ein Vernetzungsmittel und/oder ein definitionsgemässes Metallsalz enthalten, oder Gemische, die eine Verbindung der Formel I, worin R' und R" Wasserstoff, X–OH und X' Wasserstoff oder in 6-Stellung gebundenes –OH, N,N'-Diglycidyl-5,5-dimethylhydantoin, N-Glycidyl-N'-2-oxyäthylglycidyl- und/oder N-Glycidyl-N'-2-oxypropylglycidyl-5,5-dimethyl-hydantoin im Gemisch mit einer Verbindung der Formel C) und gegebenenfalls ein Vernetzungsmittel und/oder ein definitionsgemässes Metallsalz enthalten, wobei o 2–20 und z 2–11 bedeuten.

Gegenstand der Erfindung sind auch die lichtempfindlichen, gegebenenfalls vernetzten Reaktionsprodukte, die dadurch erhältlich sind, dass man gegebenenfalls in Gegenwart eines Vernetzungsmittels eine Verbindung der Formel I mit einer oder mehreren Verbindungen ausgewählt aus Di- bis Polyglycidyläthern von Phenol- und Kresolnovolacken und Verbindungen der Formeln II bis V mit geeigneten funktionellen Endgruppen und gegebenenfalls Verbindungen der Formeln VI, VII und/oder VIII zur Reaktion bringt, wobei für den Anteil an Verbindungen der Formeln VI bis VIII das oben Angegebene gilt, und die erhaltenen Reaktionsprodukte gegebenenfalls anschliessend mindestens teilweise mit einem Salz eines Metalls der Gruppe Ib oder VIII des Periodischen Systems komplexiert.

Bevorzugt sind Reaktionsprodukte, die dadurch erhältlich sind, dass man Gemische der oben angegebenen bevorzugten Art in an sich bekannter Weise zur Reaktion bringt.

Werden mehrere Verbindungen der unter 2) definierten Art eingesetzt, so kann die Umsetzung durch vorgängige (Poly)addition oder (Poly) kondensation auch stufenweise durchgeführt werden, indem z.B. das Anthrachinon der Formel I zuerst mit (einem Unter- oder Überschuss) einer ersten definitionsgemässen Reaktionskomponente umgesetzt wird und man das erhaltene Reaktionsprodukt anschliessend gegebenenfalls in Gegenwart eines Vernetzungsmittels und/oder eines definitionsgemässen Metallsalzes mit der bzw. den weiteren Reaktionskomponente(n) zur Reaktion bringt. Andererseits ist es auch möglich, zuerst verschiedene Verbindungen der unter 2) genannten Art miteinander zur Reaktion zu bringen und das erhaltene Reaktionsprodukt in einer zweiten Stufe mit dem Anthrachinon der Formel I umzusetzen.

Je nach Art der Reaktionskomponenten und der Reaktionsreihenfolge und abhängig davon, welche Reaktionskomponenten im Über- bzw. Unterschuss eingesetzt werden, können unterschiedliche Verknüpfungen des Anthrachinons mit den unter 2) genannten Verbindungen bzw. mit diesen letztgenannten Verbindungen untereinander erzielt werden. So können beispielsweise Polymere, die wiederkehrende Strukturelemente der Formel IX, X oder XI

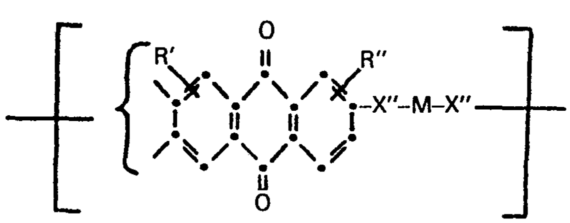

(IX),

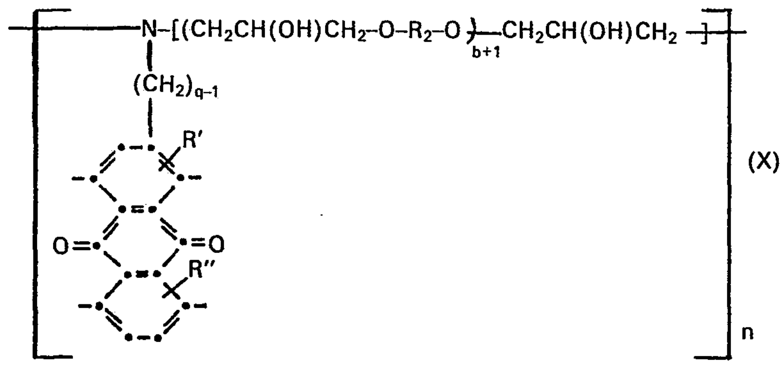

(X) oder

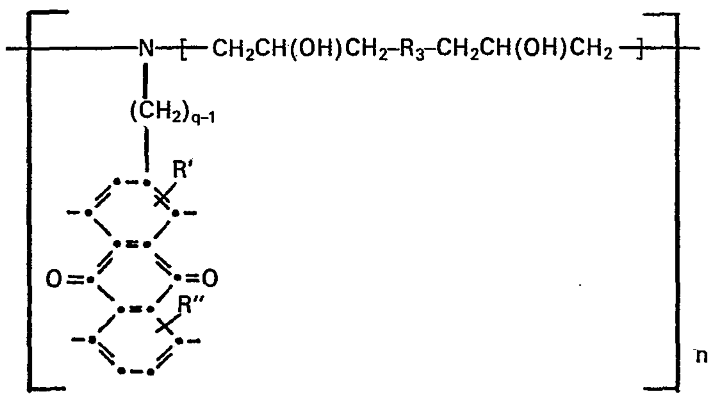

(XI)

aufweisen, oder Verbindungen der Formeln XII oder XIII hergestellt werden

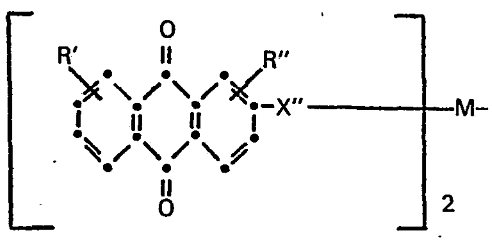

oder

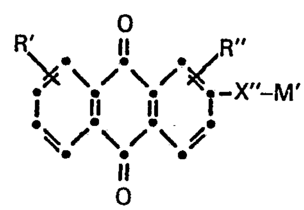

(XII) (XIII),

die mindestens teilweise mit Metallionen eines Metalls der Gruppe Ib oder VIII des Periodischen Systems komplexiert sein können, worin R' und R'' die unter Formel I angegebene Bedeutung haben,

X'' $-CO-$, $-O-C_pH_{2p}-CO-$, $-NH-$, $-N'-$, $-O-$, $-CH_2NH-$, $-CH_2N'-$, $-CH_2O-$ oder $-OCH_2CH(OH)CH_2-$ darstellt,

M, wenn X'' $-CO-$ oder $-O-C_pH_{2p}-CO-$ ist, gleiche oder verschiedene, über $-O-$ gebundene Reste von Di- bis Polyglycidyläthern von Phenol- oder Kresolnovolacken oder Gruppierungen der Formeln IIa, IIIa, IVa oder Va

$-OCH_2CH(OH)CH_2-O-CO-R_1-CO-O-CH_2CH(OH)CH_2O-$ (IIa),

$-OCH_2CH(OH)CH_2-[O-R_2-O-CH_2CH(OH)CH_2]_b-O-R_2-O-CH_2CH(OH)CH_2O-$ (IIIa),

$-OCH_2CH(OH)CH_2-R_3-CH_2CH(OH)CH_2O-$ (IVa)

oder

$-O-R_2-O-[CH_2CH(OH)CH_2-O-R_2-O]_{b'}-$ (Va)

und gegebenenfalls teilweise eine Gruppierung der Formeln VIa oder VIIIa

$-O-R-O-[CO-R_1-CO-O-R-O]_a-$ (VIa) oder

$-O-[Y_3-O]_o-Y_3-O-$ (VIIIa),

wenn X'' $-NH-$, $-O-$, $-CH_2NH-$ oder $-CH_2O-$ ist, gleiche oder verschiedene Reste von Di- bis Polyglycidyläthern von Phenol- oder Kresolnovolakken oder Gruppierungen der Formeln IIb, IIIb, oder IVb

$-CH_2CH(OH)CH_2-O-CO-R_1-CO-O-CH_2CH(OH)CH_2-$ (IIb),

$-CH_2CH(OH)CH_2-[O-R_2-O-CH_2CH(OH)CH_2]_b-$ (IIIb) oder

$-CH_2CH(OH)CH_2-R_3-CH_2CH(OH)CH_2-$ (IVb)

und gegebenenfalls teilweise eine Gruppierung der Formel VIIa

$-OC-R_1-CO-[O-R-O-CO-R_1-CO]_a-$ (VIIa),

wenn X" $-OCH_2CH(OH)CH_2-$ ist, gleiche oder verschiedene Gruppierungen der Formel Va und gegebenenfalls teilweise Gruppierungen der Formeln VIa, VIIa und/oder VIIIa, und wenn X" –N′– oder $-CH_2N'-$ ist, gleiche oder verschiedene Reste von Di- bis Polyglycidyläthern von Phenol- oder Kresolnovolacken oder Gruppierungen der Formeln IIb, IIIb oder IVb darstellt,

M′, wenn X" –CO– oder $-O-C_pH_{2p}-CO-$ ist, gleiche oder verschiedene Gruppierungen der Formeln IIIc oder Vb

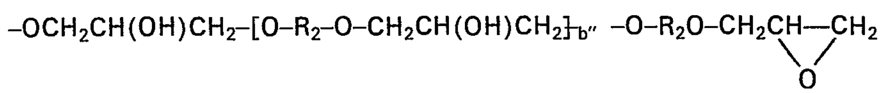

(IIIc)

oder

$-O-R_2-O-[CH_2CH(OH)CH_2-O-R_2-O]_{b''}-H$ (Vb)

und gegebenenfalls teilweise eine Gruppierung der Formeln VIb oder VIIIb

$-O-R-O-[CO-R_1-CO-O-R-O]_{a'}-H$ (VIb) oder

$-O-[Y_3-O]_{o'}-Y_3-OH$ (VIIIb),

wenn X" –NH–, –O–, $-CH_2NH-$ oder $-CH_2O-$ ist, gleiche oder verschiedene Gruppierungen der Formel IIId

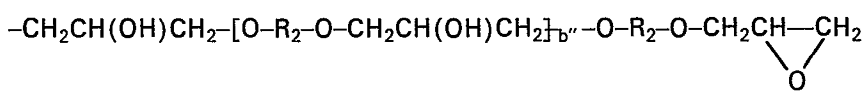

(IIId)

und gegebenenfalls teilweise eine Gruppierung der Formel VIIb

$-OC-R_1-CO-[O-R-O-CO-R_1-CO]_{a'}-OH$ (VIIb),

wenn X" $-OCH_2CH(OH)CH_2-$ ist, gleiche oder verschiedene Gruppierungen der Formel Vb und gegebenenfalls teilweise eine Gruppierung der Formeln VIb, VIIb und/oder VIIIb, und wenn X" –N′– oder $-CH_2N'-$ ist, gleiche oder verschiedene Gruppierungen der Formel IIId darstellt,

a′ eine Zahl von 5–100, b" eine Zahl von 5–150, o′ eine Zahl von 5–50 und q die Zahl 1 oder 2 bedeuten, und a, b, b′, R′, R", R, p, $R_1$, $R_2$, $R_3$ und $Y_3$ die oben angegebene Bedeutung haben, wobei X" in den Formeln XII und XIII nicht für –O– steht.

Lineare Polymere der oben angegebenen Formeln (X" ungleich –N′– oder $-CH_2N'-$) weisen bevorzugt ein durchschnittliches Molekulargewicht von 600 bis 500 000 und insbesondere von 2000 bis 150 000 Dalton auf. Das durchschnittliche Molekulargewicht der Polymeren kann nach an sich bekannten Methoden bestimmt werden, z.B. mittels Osmometrie oder Lichtstreuung.

Vernetzte Produkte können unter Umständen auch ohne Zusatz von Vernetzungsmitteln erhalten werden, z.B. bei der Umsetzung von Verbindungen der Formeln II, III, IV oder V mit entsprechenden Anthrachinonen der Formel I, insbesondere solchen, worin X bzw. X′ $-NH_2$ oder $-CH_2NH_2$ darstellen.

Die Komplexierung kann vor, nach oder bevorzugt während der Applikation der Stoffgemische bzw. der daraus erhältlichen Reaktionsprodukte erfolgen. Bei der Herstellung von erfindungsgemässen Polymeren können auch Ausgangspolymere eingesetzt werden, die definitionsgemässe Metallsalze enthalten.

Für die Komplexierung eignen sich Salze der definitionsgemässen Metalle mit organischen oder anorganischen Säuren oder Gemische davon, wie Carboxylate, z.B. Formiate, Acetate, Stearate, Gluconate und Citrate; Halogenide, Nitrate, Sulfate und Perchlorate. Als Beispiele seien genannt: Eisen(III)acetat, -citrat, -gluconat, -nitrat, -sulfat und -perchlorat; Eisen(II)- oder Eisen(III)chlorid, Eisen(II)oxalat; Ruthenium (III)chlorid; Kobalt(II)acetat, -nitrat oder -sulfat; Kobalt(II)chlorid oder -bromid; Rhodium (II)acetat, Rhodium(III)-chlorid; Nickel(II)acetat, Nickel(II)bromid und -chlorid, Nickel(II)sulfat; Palladium(II)chlorid und -jodid, Palladiumacetat und -nitrat; Kupfer(II)formiat und -acetat, Kupfer(I)- und -(II)chlorid, -bromid und -jodid, Kupfer(II)nitrat oder -sulfat; Silberacetat, -chlorid,- bromid, -nitrat oder -sulfat. Bevorzugt sind

Salze von Nicht-Edelmetallen, vor allem Eisen-, Kobalt-, Nickel- oder Kupfersalze. Ganz besonders bevorzugt sind Kupfersalze bzw. $Cu^{++}$-Ionen. Bevorzugt für die Komplexierung sind Kupfer(II)carboxylate und Kupferhalogenide. Ganz besonders verwendet man Kupfer(II)acetat oder Gemische aus Kupfer(II)acetat und Kupfer(II)bromid im molaren Verhältnis von 9:1. Der Grad der Komplexierung beträgt vorzugsweise bis zu 15%, bezogen auf die komplexierbaren Gruppen des Reaktionsprodukts bzw. der Ausgangsprodukte. Komplexierbare Gruppen sind z.B. OH–, NH– oder sekundäre Aminogruppen, wie $N(CH_3)_2$-Gruppen.

Als Vernetzungsmittel kommen je nach Art der vorhandenen funktionellen Gruppen z.B. zwei- oder höherwertige Alkohole, Phenole oder Amine, Di-, Tri- oder Tetracarbonsäuren oder Derivate davon, wie Anhydride, in Betracht. Als Beispiele geeigneter mehrfunktioneller Verbindungen seien genannt: Diole HO–R–OH oder $HO–R_2–OH$, Dicarbonsäuren $HOOC–R_1–COOH$, Oligoester der Formel VIII mit einem durchschnittlichen Molekulargewicht von 300–6000 Dalton, sowie Diamine der Formel $H_2N–R_4–NH_2$. Dabei haben R, $R_1$ und $R_2$ die oben angegebene Bedeutung und $R_4$ stellt $–C_mH_{2m}–$ mit m = 2–12, Cyclohexylen, Naphthylen, gegebenenfalls durch eine Methyl-, Methoxy- oder Nitrogruppe substituiertes Phenylen, 1,3- oder 1,4-Xylylen, den Rest des 4,4'-Diaminodicyclohexylmethans, 4,4'Diaminodiphenylmethans, 4,4'-Diaminodiphenyläthers, 4,4'-Diaminodiphenylsulfons oder Isophorondiamins dar. $R_4$ als Rest $–C_mH_{2m}–$ ist bevorzugt $–(CH_2)_2–$, Trimethylen, Tetramethylen, Hexamethylen, $–CH_2CH(CH_3)CH(CH_3)CH_2CH(CH_3)CH_2–$ oder $–CH_2C(CH_3)_2CH_2CH(CH_3)CH_2CH_2–$.

Ist $R_4$ Naphthylen oder gegebenenfalls durch eine Methyl-, Methoxy- oder Nitrogruppe substituiertes Phenylen, so kommen z.B. die folgenden Reste in Betracht: 1,2-, 1,3- und 1,4-Phenylen, 4-Methoxy-1,3-phenylen, 2-Nitro-1,4-phenylen, o- und m-Tolylen, 1,5- und 1,8-Naphthylen. Vorzugsweise stellt $R_4$ $C_mH_{2m}–$ mit m = 2–10, 1,3- oder 1,4-Phenylen oder den Rest des 4,4'-Diaminophenylmethans, 4,4'-Diamindiphenyläthers oder Isophorondiamins dar. Als Vernetzungsmittel können ferner verwendet werden: Glycerin, Tris-(hydroxymethyl)-äthan und -propan, Pentaerythrit, Diäthylentriamin, Triäthylentetramin, Bernsteinsäureanhydrid, Glutarsäureanhydrid, Phthalsäureanhydrid, Tetrahydro- und Hexahydrophthalsäureanhydrid, Trimellitsäureanhydrid, Pyromellitsäureanhydrid und Benzophenontetracarbonsäuredianhydride. Für die Vernetzung von OH– und/oder glycidylgruppenhaltigen Verbindungen verwendet man vorzugsweise Carbonsäureanhydride, wie Hexahydrophthalsäureanhydrid oder Phthalsäureanhydrid, bzw. zwei- oder mehrwertige Alkohole. Bevorzugt ist die Vernetzung von glycidylgruppenhaltigen Verbindungen mit Carbonsäureanhydriden oder zweiwertigen Alkoholen, besonders Hexahydrophthalsäureanhydrid oder Bisphenol A.

Die Kondensations- bzw. ringöffnenden Additionsreaktionen werden zweckmässig in Gegenwart eines inerten organischen Lösungsmittels bei Temperaturen zwischen 90 und 160 °C, bevorzugt 100 und 130 °C, vorgenommen. Als Lösungsmittel eignen sich z.B. Chlorbenzol, Dichlorbenzole, N,N-Dialkylamide von aliphatischen Monocarbonsäuren mit 1–3 C-Atomen im Säureteil, wie N,N-Dimethylformamid und N,N-Dimethylacetamid, Äthylenglykolmonomethyl- und -monoäthyläther, N-Methylpyrrolidon, Äthylenglykoldimethyl- oder -diäthyläther. Gegebenenfalls kann die Umsetzung in Gegenwart eines Katalysators, wie N,N-Dimethylbenzylamin, vorgenommen werden.

Gegenstand der Erfindung sind auch das neue 2-Aminomethylanthrachinon und die neuen Anthrachinone der Formel I'

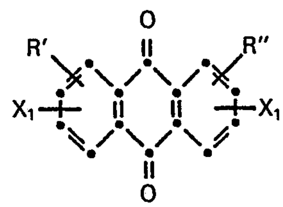
(I')

worin R' und R'' die unter Formel I angegebene Bedeutung haben, eines von $X_1$ Wasserstoff oder 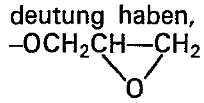 und das andere 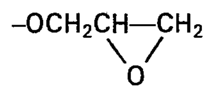 bedeutet. Dabei sind die Gruppen 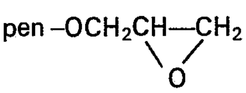 bevorzugt in 2- bzw. 2- und 6-Stellung gebunden. Die Verbindungen der Formel I' können auf an sich bekannte Weise durch Umsetzung der entsprechenden Hydroxy- bzw. Dihydroxyanthrachinone mit Epichlorhydrin hergestellt werden.

Die restlichen Verbindungen der Formel I sowie die unter 2) genannten Verbindungen sind bekannt oder können nach an sich bekannten Methoden hergestellt werden.

Die erfindungsgemässen lichtempfindlichen Stoffgemische bzw. Reaktionsprodukte finden z.B. Anwendung als Sensibilisatoren (Redox-Katalysatoren) bei verschiedenen Oxidations-/Reduktionsreaktionen oder als Beschichtungsmaterialien, z.B. für den Korrosionsschutz von Photohalbleiterdioden oder Halbleiterlaser. Besonders eignen sie sich aber zur Bildung durch Lichteinwirkung auf verschiedenen anorganischen oder organischen Substraten. Geeignete Substrate für die Bilderzeugung sind z.B. Glas, Metalle, und Metalloxide, wie Aluminium, Aluminiumoxid und Kupfer, Keramik, Papier und hochmolekulare organische Materialien. Als hochmolekulare organische Materialien kommen natürliche und synthetische Polymere in Betracht, z.B. Cellulosematerialien, wie Celluloseacetate, Cellulosepropionate,

Cellulosebutyrate und Celluloseäther, wie Methylcellulose; von α,β-ungesättigten Säuren abgeleitete Polymere, wie Polyacrylate und Polymethacrylate, Polyacrylamide und Polyacrylnitril; Styrolpolymere und Copolymere davon, z.B. Styrol/Butadiencopolymere und Acrylnitril/Butadien/Styrolcopolymere; Vinyl- und Vinylidenpolymere und Copolymere davon, wie Polyvinylchlorid, Polyvinylidenchlorid, Vinylchlorid/Vinylidenchloridcopolymere, Vinylchlorid/Vinylacetatcopolymere; von ungesättigten Alkoholen und Aminen abgeleitete Polymere und Derivate davon, wie Polyvinylalkohol, Polyvinylacetat und Polyallylmelamin; vernetzte Epoxidharze; Polyacetale; Polyalkylenoxide und Polyphenylenoxide; Polyamide, Polyimide, Polyamid/Polyimid-Blockcopolymere, Polysulfone und Polyester; Alkydharze, z.B. Glyzerin/Phthalsäureharze und deren Mischungen mit Melamin/Formaldehydharzen, Melamin-Formaldehyd-, Harnstoff-Formaldehyd-, Phenol-Formaldehydharze, usw.

Die erfindungsgemässen Stoffgemische und Reaktionsprodukte, insbesondere die Polymeren, finden vor allem Anwendung zur Erzeugung von elektrisch leitenden Überzügen oder Mustern, besonders gedruckten Schaltungen. Zu diesem Zweck werden in an sich bekannter Weise unter Lichteinwirkung die Metallionen in den Stoffgemischen oder den mindestens teilweise komplexierten Reaktionsprodukten zu nullwertigen nicht-leitenden Metallkeimen (nicht-leitenden sichtbaren Bildstellen) reduziert oder es werden bei Stoffgemischen oder Reaktionsprodukten, die keine Metallsalze bzw. Metallionen enthalten, Radikale erzeugt, auf denen dann in üblicher Weise durch stromlose Abscheidung von Metallen, wie Kupfer und Nickel usw., elektrisch leitende metallische Überzüge oder Muster hergestellt werden können. Diese metallischen Überzüge oder Muster können gewünschtenfalls durch elektrolytische Metallabscheidung mit konventionellen Metallabscheidungsbädern verstärkt werden. Für die Belichtung der erfindungsgemässen Stoffgemische oder Reaktionsprodukte können an sich beliebige geeignete Lichtquellen eingesetzt werden, z.B. Xenonlampen, Metallhalogenidlampen und besonders Quecksilberhoch- und -mitteldrucklampen.

Beispiel 1

Polymer aus 2,6-Dihydroxyanthrachinon, mit Bisphenol A vorverlängertem Bisphenol-A-diglycidylätherharz und Polyäthylenglykoldiglycidyläther.

7,21 g (0,030 Mol) 2,6-Dihydroxyanthrachinon, 8,21 g Polyäthylenglykoldiglycidyläther mit einem Epoxidäquivalent von 5,48 mAeq/g und 0,077 g N,N-Dimethylbenzylamin werden in 80 ml Äthylenglykolmonomethyläther zum Rückfluss erhitzt. Nach 4 Stunden lässt man auf Raumtemperatur abkühlen und gibt 31,76 g eines mit Bisphenol A vorverlängerten Bisphenol-A-diglycidyläthers mit einem Epoxidäquivalent von 0,629 mAeq/g zu. Anschliessend wird 5 Stunden zum Rückfluss erhitzt. Das erhaltene Polymer wird in Wasser gefällt und im Vakuum bei 80 °C getrocknet. Ausbeute 43,5 g (92% d.Th.). Tg = 44 °C; durchschnittliches Molekulargewicht (mittels Osmometrie gemessen) = 3500 Dalton. Grenzviskosität [η] = 0,134 dl/g (0,5 Gew.-% bei 25 °C in Äthylenglykolmonomethyläther).

Beispiel 2

Polymer aus 2,6-Dihydroxyanthrachinon, Polypropylenglykoldiglycidyläther und mit Bisphenol A vorverlängertem Bisphenol-A-diglycidylätherharz.

3,6 g (0,015 Mol) 2,6-Dihydroxyanthrachinon, 7,56 g eines Polypropylenglykoldiglycidyläthers mit einem Epoxidäquivalentgewicht von 2,639 mAeq/g und 0,6 g N,N-Dimethylbenzylamin werden in 50 ml Äthylenglykolmonoäthyläther zum Rückfluss erhitzt. Nach 8 Stunden wird auf Raumtemperatur abgekühlt. Dann werden 23,82 g eines mit Bisphenol A vorverlängerten Bisphenol-A-diglycidyläthers mit einem Epoxidäquivalent von 0,629 mAeq/g zugegeben und erneut während 5 Stunden zum Rückfluss erhitzt. Das resultierende Polymer wird aus Wasser gefällt und im Vakuum bei 80 °C getrocknet. Ausbeute 26,5 g; Epoxidgehalt 0,085 mAeq/g; durchschnittliches Molekulargewicht 4200 Dalton (bestimmt durch Osmometrie).

Beispiel 3

Polymer aus 2,6-Dihydroxyanthrachinon, Hydantoindiglycidyläther und vorverlängertem Bisphenol-A-diglycidylätherharz. 7,21 g (0,03 Mol) 2,6-Dihydroxyanthrachinon und 6,25 g eines Gemisches aus 70 Gew.-teilen N,N'-Diglycidyl-5,5-dimethylhydantoin und 30 Gew.-teilen N-Glycidyl-N'-oxypropyl-glycidyl- 5,5-dimethylhydantoin werden in 80 ml Äthylenglykolmonoäthyläther mit 0,067 g N,N-Dimethylbenzylamin versetzt und 4 Std. am Rückfluss gehalten. Anschliessend wird auf 50 °C abgekühlt. Dann gibt man 31,67 g eines mit Bisphenol A vorverlängerten Bisphenol-A-diglycidyläthers mit einem Epoxidäquivalent von 0,629 mAeq/g zu und erhitzt erneut 4 Std. am Rückfluss. Man erhält eine dunkelrote, viskose Lösung, aus der das Polymer durch Fällen aus Wasser erhalten wird. Ausbeute 45,1 g; Tg = 50 °C (bestimmt durch Differential Scanning Calorimetry DSC bei 20 °C/Min.). Durchschnittliches Molekulargewicht 6000 Dalton (bestimmt durch Gelpermeationschromatographie). Epoxidäquivalent 0,04 mAeq/g.

Beispiel 4

Analog den in den Beispielen 1–3 beschriebenen Verfahren wird aus 0,05 Mol 2,6-Dihydroxyanthrachinon und 30,73 g eines Polypropylenglykoldiglycidyläthers mit einem Epoxidäquivalent von 3,25 mAeq/g ein vorverlängertes Produkt hergestellt (Epoxidäquivalent 0,377 mAeq/g). 26,5 g dieses Produkts werden mit 15,88 g eines mit Bisphenol A vorverlängerten Bisphenol-A-diglycidyläthers mit einem Epoxidäquivalent von 0,629 mAeq/g, 0,771 g Hexahydrophthalsäureanhydrid und 2,16 g Kupfer(II)

acetat in 200 ml N,N-Dimethylformamid gelöst. Mit dieser Lösung werden auf Polyesterfolien oder auf glasfaserverstärkten Epoxidplatten Filme hergestellt und durch 4stündiges Trocknen bei 120 °C vernetzt.

Beispiel 5

20,77 g (0,09 Mol) Bisphenol A, 27,04 g (0,078 Mol) Bisphenol-A-diglycidyläther und 0,239 g N,N-Dimethylbenzylamin werden in 50 ml Äthylenglykolmonomethyläther unter Inertgas und Rühren auf 125 °C Innentemperatur erhitzt und 3 Stunden lang gerührt. Die Bestimmung der phenolischen OH-Gruppen ergibt 0,676 mAeq/g. Nach dem Abkühlen auf Raumtemperatur werden 2,96 g eines Polyäthylenglykoldiglycidyläthers mit einem Epoxidäquivalent von 5,48 mAeq/g und 3,78 g 2,6-Anthrachinondiglycidyläther zugegeben. Dann wird das Reaktionsgemisch mit 50 ml Äthylenglykolmonoäthyläther verdünnt und erneut während 3 Stunden auf 125 °C erhitzt. Das Polymer wird durch Fällen in Wasser isoliert. Ausbeute: 50,2 g (92% d.Th.); Tg = 49 °C; durchschnittliches Molekulargewicht 7300 Dalton (bestimmt durch Osmometrie).

Beispiel 6–8

Analog den in den Beispielen 1–3 beschriebenen Verfahren werden Polyethylenglykoldiglycidyläther mit einem Epoxidäquivalentgewicht von 5,48 mAeq/g sowie ein mit Bisphenol A vorverlängerter Bisphenol-A-diglycidyläther mit einem Epoxidäquivalentgewicht von 0,629 mAeq/g in einem Gemisch aus 6 Teilen Dioxan und 4 Teilen N-Methylpyrrolidon zusammen mit 2,6-Anthrachinonyldi(oxoessigsäure) gelöst und mit 0,5 Gew.-% Dimethylbenzylamin versetzt.

Die Lösung wird 4 Stunden bei 100 °C gerührt. Man lässt dann abkühlen und setzt 2,5 Gew.-% Kupferacetat zu. Analog dem Verfahren in Beispiel 9 werden Filme auf Glasplatten hergestellt, bei 80 °C 1 Stunde getrocknet und dann 30 Minuten bei 180 °C gehärtet.

Die eingesetzten Mengen und die Glasumwandlungstemperatur ergeben sich aus nachfolgender Tabelle.

| Beispiel Nr. | 2,6-Antrachinonyldioxoessigsäure/g | Polyethylenglykoldiglycidyläther/g | Bisphenol-A-bisphenol-A-diglycidylätheraddukt/g | Tg/ °C |
|---|---|---|---|---|
| 6 | 2,00 | 1,573 | 1,626 | 38 |
| 7 | 2,00 | 1,416 | 1,940 | 52 |
| 8 | 2,00 | 0,885 | 7,701 | 62 |

Beispiel 9

Zur Prüfung der Lichtempfindlichkeit werden jeweils 6 g der in der folgenden Tabelle angegebenen Polymeren in 20 ml N,N-Dimethylformamid gelöst und mit 110 mg Kupfer(II)acetat und 10 mg $CuBr_2$ versetzt. Diese Lösung wird mit einem Rakelstab auf eine Polyesterfolie aufgetragen (Nassfilmdicke 50 μm) und nach Ablüften bei Raumtemperatur und Trocknen bei 70 °C in einem Umluftofen durch eine Maske (21-Step Sensitivity Guide der Fa. Stouffer) mit einer 5 kW Quecksilberhochdrucklampe belichtet. Das nach dem Belichten sichtbare Bild wird in einem Kupferbad der Zusammensetzung $CuSO_4 \cdot 5H_2O$ 12 g/l, HCOH 8 g/l, NaOh 15 g/l, Natriumkaliumtartrat 14 g/l, Äthylendiamintetraessigsäure 20 g/l, Octylphenolpolyäthylenglykoläther 1 gl bei 49 °C weiter verstärkt. Die Resultate sind in der folgenden Tabelle angegeben.

| Polymer nach Beispiel | Belichtungszeit Min. | Belichtungstemperatur °C | letzte abgebildete Stufe |
|---|---|---|---|
| 1 | 3 | 90 | 3 |
| 2 | 6 | 90 | 4 |
| 3 | 3 | 85 | 2 |
| 4 | 1,5 | 90 | 1 |
| 5*) | 3 | 90 | 3 |
| 7 | 3 | 90 | 4 |
| 8 | 3 | 90 | 4 |
| 9 | 6 | 90 | 1 |

*) 5 Gew.-% Kupfer(II)acetat.

Herstellung von neuen Anthrachinonen:

Beispiel A

Herstellung von 2-Anthrachinonylglycidyläther

112,1 g (0,5 Mol) 2-Hydroxyanthrachinon werden in 500 ml N-Methylpyrrolidon gelöst und portionenweise mit 12 g (0,5 Mol) Natriumhydrid versetzt. Nach Beendigung der Gasentwicklung werden bei 70 °C 46,37 ml (0,5 Mol) Epichlorhydrin zugetropft und noch 8 Stunden bei 70 °C gerührt. Anschliessend versetzt man mit 1,5 l Wasser, filtriert ab und trocknet den Filterkuchen im Vakuum. Nach dem Trocknen wird das Produkt erneut in 1 l N,N-Dimethylformamid mit 10 g Aktivkohle aufgekocht und heiss filtriert. Die beim Abkühlen auf 0 °C ausfallenden Kristalle werden durch Filtration abgetrennt und im Hochvakuum getrocknet. Man erhält 69,45 g (50,57% d.Th.) 2-Anthrachinonylglycidyläther; Smp. 162–167 °C.

Elementaranalyse:

| | | | |
|---|---|---|---|
| berechnet | C 72,85 | H 4,32 | O 22,83% |
| gefunden | C 73,0 | H 4,1 | O 22,2%. |

Beispiel B

Herstellung von 2,6-Anthrachinondiglycidyläther.

30,0 g (0,125 Mol) 2,6-Dihydroxyanthrachinon werden in 300 ml N-Methylpyrrolidon bei 50 °C gelöst und portionenweise mit 0,25 Mol Natriumhydrid (als Suspension in Öl) versetzt. Nach Beendigung der Gasentwicklung werden 35 g Epichlorhydrin zugegeben und 20 Std. bei 80 °C gerührt. Die Lösung wird in 5 Liter Eiswasser gegeben, und das ausgefallene Produkt wird abfiltriert und getrocknet. Man erhält 38,3 g (97% d.Th.) 2,6-Anthrachinondiglycidyläther. Die Umkristallisation aus Äthylenglykolmonomethyläther mit 3 Gew.-% Aktivkohle liefert 22,1 g Produkt; Smp. 203 °C.

Elementaranalyse:

| | | | |
|---|---|---|---|
| berechnet | C 68,18 | H 4,58 | O 27,25% |
| gefunden | C 68,05 | H 4,68 | O 27,46%. |

Beispiel C

Herstellung von 2-Aminomethylanthrachinon

10 g (0,039 Mol) 2-Chlormethylanthrachinon [hergestellt nach G. Izoret, Ann. Chim., 7, 180 (1962)] werden in 50 ml N,N-Dimethylformamid (DMF) gelöst und unter Rühren mit 2,53 g (0,039 Mol) Natriumazid versetzt. Nach 1 Stunde wird die Temperatur auf 50 °C erhöht und 10 Stunden bei dieser Temperatur gerührt. Man versetzt mit 250 ml Wasser und filtriert ab. Man erhält 9,5 g (92,3% d.Th.) 2-Azidomethyl-anthrachinon; Smp. 130 °C (Zersetzung).

Analyse für $C_{15}H_9O_2N_3$:

| | | | |
|---|---|---|---|
| berechnet | C 68,44 | H 3,45 | N 15,96% |
| gefunden | C 67,39 | H 3,41 | N 15,60%. |

9,5 g 2-Azidomethylanthrachinon werden in 100 ml DMF gelöst und mit 1,0 g Platin/Kohle-Katalysator (5 Gew.-% Pt) versetzt und mit Wasserstoff 15 Minuten hydriert. Der Katalysator wird abfiltriert, das Filtrat wird mit 200 ml Wasser versetzt und filtriert. Die ausgefallenen roten Kristalle werden aus Dioxan umkristallisiert. Man erhält 6,5 g (70,01% d.Th.) 2-Aminomethylanthrachinon; Smp. 170–78 °C.

Analyse für $C_{15}H_{11}O_2N$:

| | | | |
|---|---|---|---|
| berechnet | C 75,94 | H 4,67 | N 5,91% |
| gefunden | C 74,23 | H 4,65 | N 5,74%. |

D) Herstellung von 2,6-Anthrachinonyldioxoessigsäure

0,125 Mol (30 g) 2,6-Dihydroxyanthrachinon werden unter Inertgas und Rühren in 500 ml N-Methylpyrrolidon gelöst und portionenweise mit 0,25 Mol Natriumhydrid versetzt. Nach Beendigung der Wasserstoffentwicklung werden 0,5 Mol (61,2 g) Chloressigsäureethylester zugegeben und 2 Stunden bei 100 °C gerührt. Man gibt 2 l Wasser zu, stellt mit HCl neutral und trennt das ausgefallene Produkt durch Filtration ab. Ausbeute nach Umkristallisation aus Essigester 31 g = 60,1% der Theorie. Smp. 175 °C.

30 g des Dicarbonsäureesters werden in Oleum gelöst und in Wasser eingetropft. Das ausgefallene Produkt wird durch Filtration abgetrennt.

Ausbeute: 19,4 g = (72% der Theorie) Smp.: > 300 °C. Säuregehalt: 97,15%.

## Patentansprüche

1. Zu Kondensations- oder Additionsreaktionen befähigte lichtempfindliche und gegebenenfalls vernetzbare Stoffgemische, enthaltend

1) ein Anthrachinon der Formel I

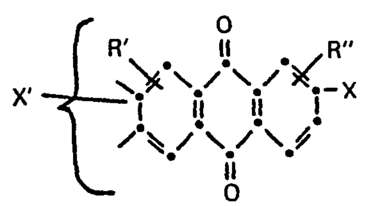

(I),

2) eine oder mehrere Verbindungen ausgewählt aus Di- bis Polyglycidyläthern von Phenol- und Kresolnovolacken und Verbindungen der Formeln II bis VIII

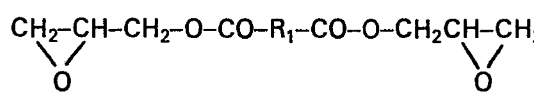

(II),

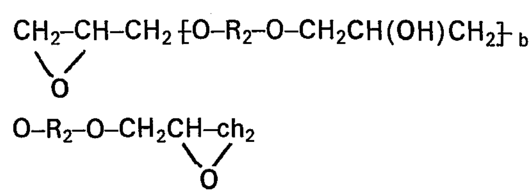

III),

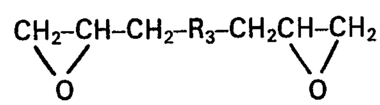

(IV),

$HO{-}R_2{-}O\,[CH_2CH(OH)CH_2{-}O{-}R_2{-}O]_{b'}\,{-}H$ (V),

$HO{-}R{-}O\,[CO{-}R_1{-}CO{-}O{-}R{-}O]_a{-}H$ (VI),

$HOOC-R_1-CO\{O-R-O-CO-R_1-CO\}_aOH$ (VII)

und

$HO\{Y_3-O\}_o-Y_3-OH$ (VIII),

wobei der Anteil an Verbindungen der Formeln VI, VII und/oder VIII höchstens 80 Mol%, bezogen auf alle unter 2) genannten Reaktionskomponenten beträgt,

3) gegebenenfalls ein Vernetzungsmittel und

4) gegebenenfalls ein Salz eines Metalls der Gruppe Ib oder VIII des Periodischen Systems, worin

R' und R'' unabhängig voneinander Wasserstoff, Methyl, Halogen, eine Nitro-, Phenylsulfonyl- oder Methoxygruppe bedeuten,

X $-COOH$, $-COCl$, $-O-C_pH_{2p}-COOH$ mit p = 1 bis 4, $-NH_2$, $-OH$, $-CH_2NH_2$, $-CH_2OH$ oder $-OCH_2CH-CH_2$ darstellt,
O

X' Wasserstoff bedeutet oder dieselbe Bedeutung wie X hat, mit Ausnahme von Hydroxyanthrachinon,

a eine Zahl von 1–100,

b eine Zahl von 0–150,

b' eine Zahl von 0,1–150,

R $-C_mH_{2m}-$ mit m = 2–12, $\{CH_2CH_2O\}_r-CH_2CH_2-$ mit r = 1–40, $-CH(CH_3)CH_2OCH_2CH(CH_3)-$, Cyclohexylen,

$-CH_2-C(CH_3)_2-OCOC(CH_3)_2CH_2-$, 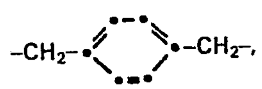

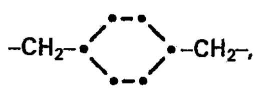 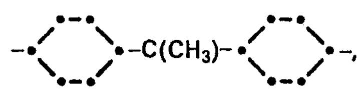

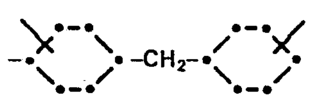 ,

Naphthylen, Biphenylen oder gegebenenfalls durch eine Methyl-, Methoxy- oder Nitrogruppe substituiertes Phenylen,

$R_1$ die direkte Bindung, $-C_mH_{2m}-$ mit m = 2–12 oder Cyclohexylen, Cyclohexenylen, Phenylen oder Endomethylencyclohexenylen, die durch eine Methylgruppe substituiert sein können,

$R_2$ $-C_mH_{2m}-$ mit m = 2–12, Phenylen,

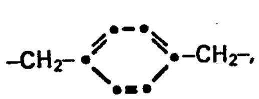 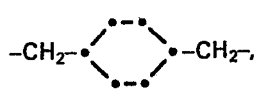

eine Gruppe der Formeln

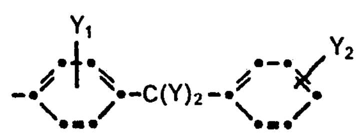 oder $-(Y_3O)_o-Y_3-$ ,

Y Wasserstoff oder Methyl,

$Y_1$ und $Y_2$ unabhängig voneinander Wasserstoff, Chlor oder Brom,

$Y_3$ $-(CH_2)_2-$, $-CH_2CH(CH_3)-$ oder $-(CH_2)_4-$,

o eine Zahl von 1–50 und

$R_3$ 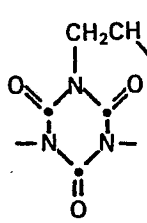 , 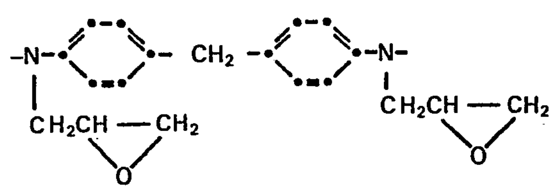

den Rest des Äthylenharnstoffs, 1,3-Propylenharnstoffs, 5,5-Dimethylhydantoins, 2-Hydroxyäthyl-5,5-dimethylhydantoins oder 2-Hydroxypropyl-5,5-dimethylhydantoins bedeuten.

2. Stoffgemische nach Anspruch 1, enthaltend ein Anthrachinon der Formel I, eine oder mehrere Verbindungen mit Glycidylendgruppen und/oder eine Verbindung der Formel V, sowie gegebenenfalls ein Vernetzungsmittel und/oder ein definitionsgemässes Metallsalz, worin R' und R'' Wasserstoff sind, X $-CH_2NH_2$, $-O-C_pH_{2p}-COOH$ oder $-OH$ und X' Wasserstoff oder in 6-Stellung gebundenes $-O-C_pH_{2p}-COOH$ oder $-OH$, b' eine Zahl von 0,1–100 und b eine Zahl von 1–100 bedeuten.

3. Stoffgemische nach Anspruch 1, enthaltend ein Anthrachinon der Formel I, worin R' und R'' Wasserstoff, X $-CH_2NH_2$ oder $-OH$ und X' Wasserstoff oder in 6-Stellung gebundenes $-OH$ bedeuten, eine oder mehrere Verbindungen ausgewählt aus Di- und/oder Triglycidyläthern von

Phenol- oder Kresolnovolacken, Triglycidylisocyanurat, Hexahydrophthalsäurediglycidylester, N,N'-Diglycidyl-5,5-dimethylhydantoin, N-Glycidyl-N'-2-oxyäthylglycidyl- und/oder N-Glycidyl-N'-oxypropylglycidyl- 5,5-dimethylhydantoin und Verbindungen der Formeln A), B), C) und D)

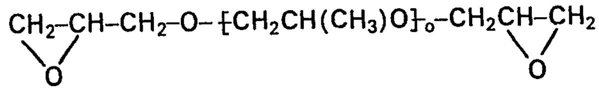

(A)

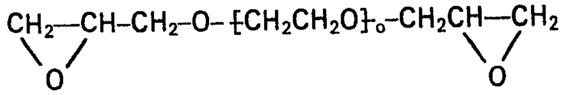

(B),

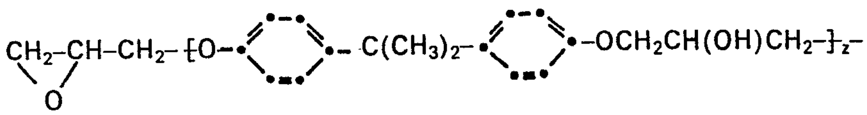

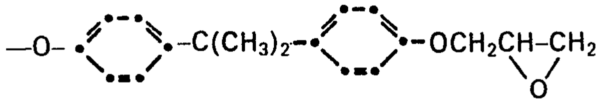

(C)

und

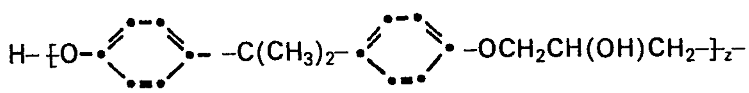

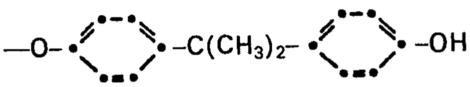

(D),

sowie gegebenenfalls ein Vernetzungsmittel und/ oder ein definitionsgemässes Metallsalz, wobei o 2–40 und z 0,1–13 bedeuten.

4. Stoffgemische nach Anspruch 1, enthaltend ein Anthrachinon der Formel I, worin R' und R" Wasserstoff, X –OH und X' Wasserstoff oder in 6-Stellung gebundenes –OH darstellen, eine Verbindung der Formel A) oder B)

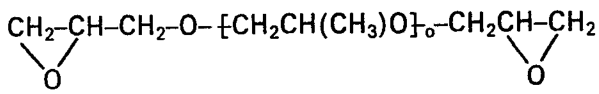

(A) oder

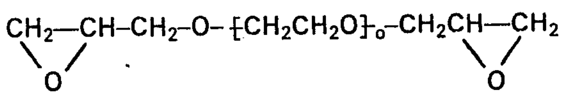

(B)

im Gemisch mit einer Verbindung der Formel C)

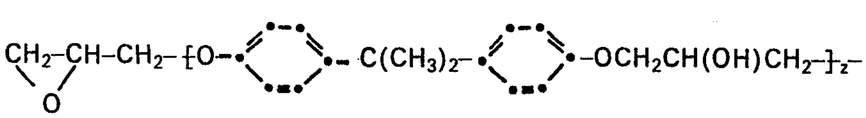

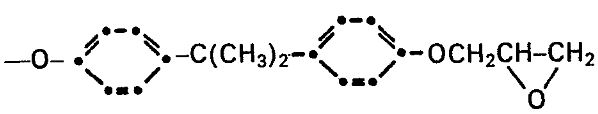

(C),

sowie gegebenenfalls ein Vernetzungsmittel und/ oder ein definitionsgemässes Metallsalz, wobei o 2–20 und z 2–11 bedeuten.

5. Stoffgemische nach Anspruch 1, enthaltend ein Anthrachinon der Formel I, worin R' und R" Wasserstoff, X –OH und X' Wasserstoff oder in 6-Stellung gebundenes –OH darstellen, N,N'-Diglycidyl-5,5-dimethylhydantoin, N-Glycidyl-N'-2-oxyäthyl- und/oder N-Glycidyl-N'-2-oxypropylglycidyl-5,5-dimethylhydantoin im Gemisch mit einer Verbindung der Formel C)

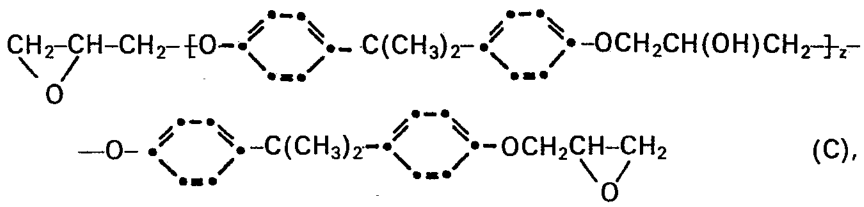

(C),

sowie gegebenenfalls ein Vernetzungsmittel und/oder ein definitionsgemässes Metallsalz, wobei o 2–20 und z 2–11 bedeuten.

6. Stoffgemische nach Anspruch 1, die ein Eisen-, Kobalt-, Nickel- oder Kupfersalz enthalten.

7. Stoffgemische nach Anspruch 1, die Kupfer(II)acetat oder ein Gemisch aus Kupfer(II)acetat und Kupfer(II)bromid enthalten.

8. Stoffgemische nach Anspruch 1, die als Vernetzungsmittel Hexahydrophthalsäureanhydrid oder Bisphenol A enthalten.

9. Lichtempfindliche, gegebenenfalls vernetzte Reaktionsprodukte, die dadurch erhältlich sind, dass man gegebenenfalls in Gegenwart eines Vernetzungsmittels eine Verbindung der Formel I mit einer oder mehreren Verbindungen ausgewählt aus Di- bis Polyglycidyläthern von Phenol- oder Kresolnovolacken und Verbindungen der Formeln II bis V mit geeigneten funktionellen Endgruppen und gegebenenfalls Verbindungen der Formeln VI, VII und/oder VIII gemäss Anspruch 1 zur Reaktion bringt, wobei der Anteil an Verbindungen der Formel VI bis VIII höchstens 80 Mol%, bezogen auf alle im Anspruch 1 unter 2) angegebenen Verbindungen beträgt, und die erhaltenen Reaktionsprodukte gegebenenfalls anschliessend mindestens teilweise mit einem Salz eines Metalls der Gruppe Ib oder VIII des Periodischen Systems komplexiert.

10. Lichtempfindliche Reaktionsprodukte nach Anspruch 9, dadurch gekennzeichnet, dass man die erhaltenen Reaktionsprodukte mindestens teilweise mit einem Eisen-, Kobalt-, Nickel- oder Kupfersalz komplexiert.

11. Lichtempfindliche Reaktionsprodukte nach Anspruch 9, dadurch gekennzeichnet, dass man die erhaltenen Reaktionsprodukte mindestens teilweise mit Kupfer(II)acetat oder einem Gemisch aus Kupfer(II)acetat und Kupfer(II)bromid komplexiert.

12. Lichtempfindliche Reaktionsprodukte nach Anspruch 9, dadurch gekennzeichnet, dass man als Vernetzungsmittel Hexahydrophthalsäureanhydrid oder Bisphenol A verwendet.

13. Lichtempfindliche Reaktionsprodukte nach Anspruch 9, dadurch gekennzeichnet, dass sie Polymere mit wiederkehrenden Strukturelementen der Formeln IX, X oder XI

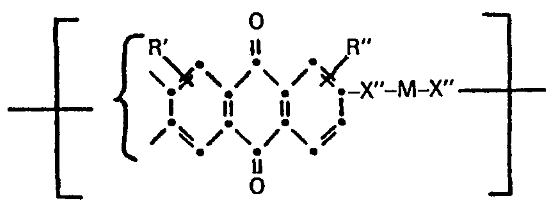

(IX),

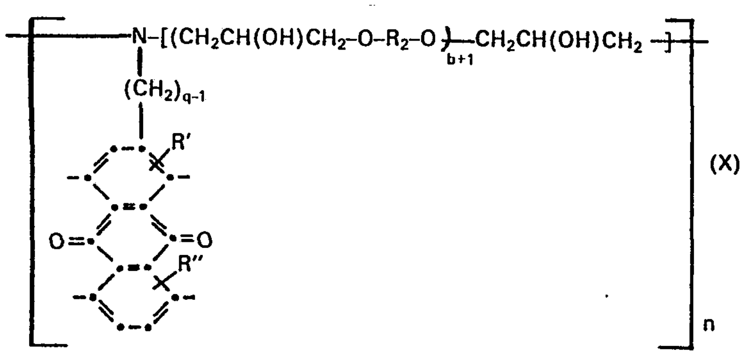

(X) oder

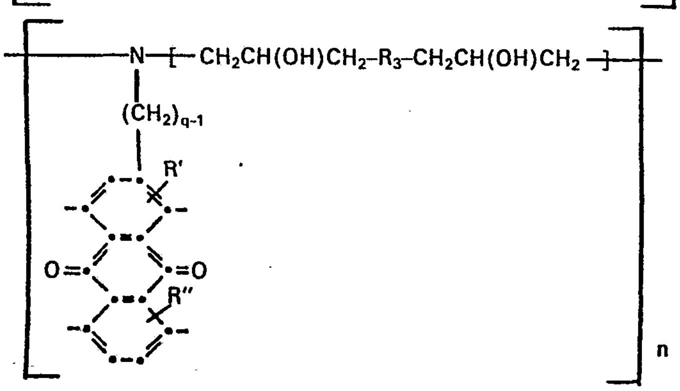

(XI)

oder Verbindungen der Formel XII oder XIII sind,

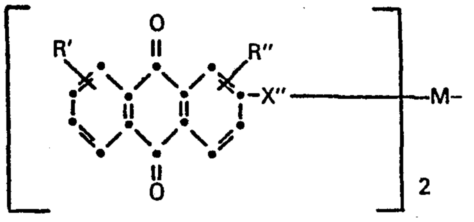 oder 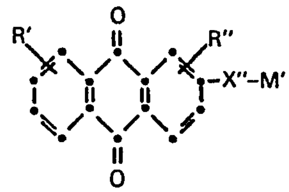

(XII) (XIII),

die mindestens teilweise mit Metallionen eines Metalls der Gruppe Ib oder VIII des Periodischen Systems komplexiert sein können, worin R' und R'' unabhängig voneinander Wasserstoff, Methyl, Halogen, eine Nitro-, Phenylsulfonyl- oder Methoxygruppe bedeuten, X'' $-CO-$, $-O-C_pH_{2p}-CO-$ mit p = 1 bis 4, $-NH-$, $-\overset{|}{N}'-$, $-O-$, $-CH_2NH-$, $-CH_2\overset{|}{N}'-$, $-CH_2O-$ oder $-OCH_2CH(OH)CH_2-$ darstellt, M, wenn X'' $-CO$ oder $-O-C_pH_{2p}-CO-$ ist, gleiche oder verschiedene, über $-O-$ gebundene Reste von Di- bis Polyglycidyläthern von Phenol- oder Kresolnovolacken oder Gruppierungen der Formeln IIa, IIIa, IVa oder Va

$$-OCH_2CH(OH)CH_2-O-CO-R_1-CO-O-CH_2CH(OH)CH_2O- \quad (IIa),$$

$$-OCH_2CH(OH)CH_2-[O-R_2-O-CH_2CH(OH)CH_2]_b-O-R_2-O-CH_2CH(OH)CH_2O- \quad (IIIa),$$

$$-OCH_2CH(OH)CH_2-R_3-CH_2CH(OH)CH_2O- \quad (IVa)$$

oder

$$-O-R_2-O-[CH_2CH(OH)CH_2-O-R_2-O]_{b'}- \quad (Va)$$

und gegebenenfalls teilweise eine Gruppierung der Formeln VIa oder VIIIa

$$-O-R-O-[CO-R_1-CO-O-R-O]_a- \quad (VIa)$$

oder $-O-[Y_3-O]_o-Y_3-O-$ (VIIIa),

wenn X'' $-NH-$, $-O-$, $-CH_2NH-$ oder $-CH_2O-$ ist, gleiche oder verschiedene Reste von Di- bis Polyglycidyläthern von Phenol- oder Kresolnovolacken oder Gruppierungen der Formeln IIb, IIIb oder IVb

$$-CH_2CH(OH)CH_2-O-CO-R_1-CO-O-CH_2CH(OH)CH_2- \quad (IIb),$$

$$-CH_2CH(OH)CH_2-[O-R_2-O-CH_2CH(OH)CH_2]_b- \quad (IIIb) \text{ oder}$$

$$-CH_2CH(OH)CH_2-R_3-CH_2CH(OH)CH_2- \quad (IVb)$$

und gegebenenfalls teilweise eine Gruppierung der Formel VIIa

$$-OC-R_1-CO-[O-R-O-CO-R_1-CO]_a- \quad (VIIa),$$

wenn X'' $-OCH_2CH(OH)CH_2-$ ist, gleiche oder verschiedene Gruppierungen der Formel Va und gegebenenfalls teilweise Gruppierungen der Formeln VIa, VIIa und/oder VIIIa, und wenn X'' $-\overset{|}{N}'-$ oder $-CH_2\overset{|}{N}'-$ ist, gleiche oder verschiedene Reste von Di- bis Polyglycidyläthern von Phenol- oder Kresolnovolacken oder Gruppierungen der Formeln IIb, IIIb oder IVb darstellt,

M', wenn X'' $-CO-$ oder $-O-C_pH_{2p}-CO-$ ist, gleiche oder verschiedene Gruppierungen der Formeln IIIc oder Vb

$$-OCH_2CH(OH)CH_2-[O-R_2-O-CH_2CH(OH)CH_2]_{b''}-O-R_2O-CH_2\underset{\diagdown O \diagup}{CH-CH_2} \quad (IIIc)$$

oder

$$-O-R_2-O-[CH_2CH(OH)CH_2-O-R_2-O]_{b''}-H \quad (Vb)$$

und gegebenenfalls teilweise eine Gruppierung der Formeln VIb oder VIIIb

–O–R–O–[CO–R₁–CO–O–R–O]$_{a'}$ –H (VIb) oder

–O–[$Y_3$–O]$_{o'}$ –$Y_3$–OH (VIIIb),

wenn X'' –NH–, –O–, –$CH_2$NH– oder –$CH_2$O– ist, gleiche oder verschiedene Gruppierungen der Formel IIId

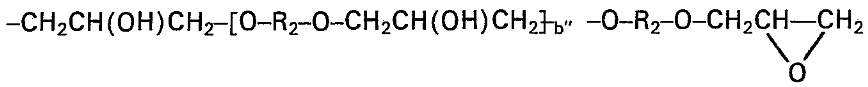

(IIId)

und gegebenenfalls teilweise eine Gruppierung der Formel VIIb

–OC–$R_1$–CO–[O–R–O–CO–$R_1$–CO]$_{a'}$ –OH (VIIb),

wenn X'' –$OCH_2CH(OH)CH_2$– ist, gleiche oder verschiedene Gruppierungen der Formel Vb und gegebenenfalls teilweise eine Gruppierung der Formeln VIb, VIIb und/oder VIIIb, und wenn X'' –N'– oder –$CH_2$–N'– ist, gleiche oder verschiedene Gruppierungen der Formel IIId darstellt, a eine Zahl von 1–100, b eine Zahl von 0–150, b' eine Zahl von 0,1–150, q die Zahl 1 oder 2, b'' eine Zahl von 5–150, a' eine Zahl von 5–100, o' eine Zahl von 5–50,

R –$C_mH_{2m}$ mit m = 2–12, –[$CH_2CH_2O$)$_r$–$CH_2CH_2$– mit r = 1–40, –$CH(CH_3)CH_2OCH_2CH(CH_3)$–, Cyclohexylen,

–$CH_2$–$C(CH_3)_2$–$OCOC(CH_3)_2CH_2$–, 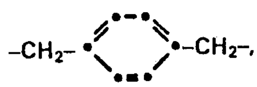

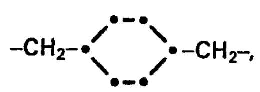 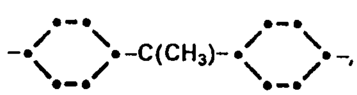

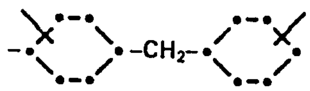 ,

Naphthylen, Biphenylen oder gegebenenfalls durch eine Methyl-, Methoxy- oder Nitrogruppe substituiertes Phenylen, $R_1$ die direkte Bindung, –$C_mH_{2m}$– mit m = 2–12 oder Cyclohexylen, Cyclohexenylen, Phenylen oder Endomethylencyclohexenylen, die durch eine Methylgruppe substituiert sein können,

$R_2$ –$C_mH_{2m}$– mit m = 2–12, Phenylen,

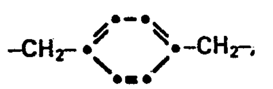 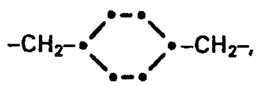

eine Gruppe der Formeln

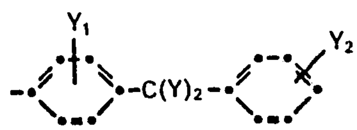 oder -$(Y_3O)_o$–$Y_3$– ,

Y Wasserstoff oder Methyl,

$Y_1$ und $Y_2$ unabhängig voneinander Wasserstoff, Chlor oder Brom,

$Y_3$ –$(CH_2)_2$–, –$CH_2CH(CH_3)$– oder –$(CH_2)_4$–,

o eine Zahl von 1–50 und

$R_3$ 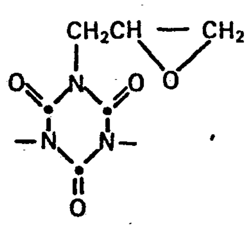 , 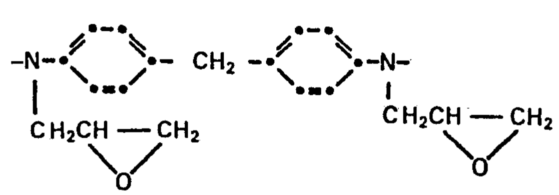

den Rest des Äthylenharnstoffs, 1,3-Propylenharnstoffs, 5,5-Dimethylhydantoins, 2-Hydroxyäthyl-5,5-dimethylhydantoins oder 2-Hydroxypropyl-5,5-dimethylhydantoins bedeuten, wobei X'' in den Formeln XII und XIII nicht für –O– steht.

14. Lichtempfindliche Reaktionsprodukte nach Anspruch 13, die mindestens teilweise mit Eisen-, Kobalt-, Nickel- oder Kupferionen komplexiert sind.

15. Lichtempfindliche Reaktionsprodukte nach Anspruch 13, die mindestens teilweise mit $Cu^{++}$-Ionen komplexiert sind.

16. Anthrachinone der Formel I'

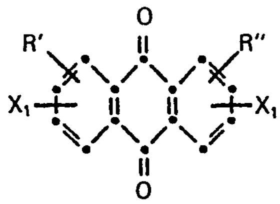
(I')

worin R' und R'' unabhängig voneinander Wasserstoff, Methyl, Halogen, eine Nitro-, Phenylsulfonyl- oder Methoxygruppe, eines von $X_1$ Wasserstoff oder 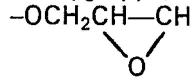 und das andere 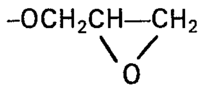 bedeuten, oder R' und R'' ein Wasserstoffatom, ein $X_1$ ein Wasserstoffatom und das andere in 2-Stellung gebundenes Aminomethyl bedeuten.

17. Anthrachinone der Formel I' nach Anspruch 16, worin die Gruppen 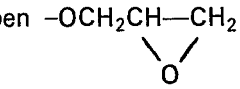 in 2- bzw. in 2- und 6-Stellung gebunden sind.

18. Verwendung von Stoffgemischen nach Anspruch 1, die kein definitionsgemässes Metallsalz enthalten, oder von Polymeren oder Verbindungen nach Anspruch 13, die nicht mit Metallionen eines Metalls der Gruppen Ib oder VIII des Periodischen Systems komplexiert sind, zur Herstellung von elektrisch leitenden Überzügen oder Mustern durch Belichtung und stromlose Abscheidung von Metallen.

**Revendications**

1. Mélanges photosensibles, capables de donner des réactions de condensation ou d'addition et éventuellement réticulables, mélanges qui contiennent:

1) une anthraquinone de formule I:

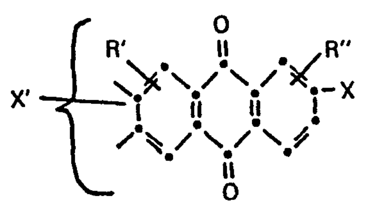
(I),

2) un ou plusieurs composés pris dans l'ensemble constitué par les éthers diglycidyliques et polyglycidyliques de novolaques phénoliques et crésoliques et les composés répondant aux formules II à VIII:

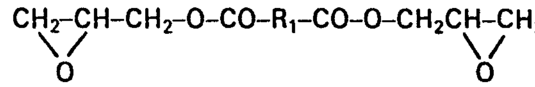
(II),

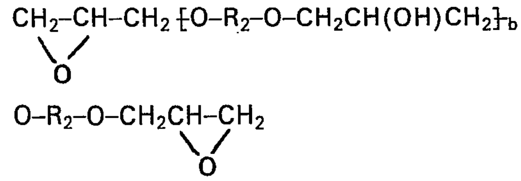
III),

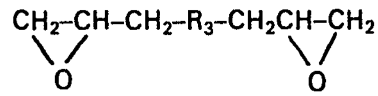
(IV),

$HO–R_2–O\{CH_2CH(OH)CH_2–O–R_2–O\}_{b'}–H$ (V),

$HO–R–O\{CO–R_1–CO–O–R–O\}_a–H$ (VI),

$HOOC–R_1–CO\{O–R–O–CO–R_1–CO\}_aOH$ (VII),

$HO\{Y_3–O\}_o–Y_3–OH$ (VIII),

la proportion des composés de formules VI, VII et/ou VIII étant au plus égale à 80% en moles par rapport à toutes les composantes réactionnelles mentionnées sous 2),

3) éventuellement un réticulant et

4) éventuellement un sel d'un métal du groupe Ib ou VIII de la classification périodique, les symboles présents dans les formules représentées ci-dessus ayant les significations suivantes:

R' et R'' représentent chacun, indépendamment l'un de l'autre, l'hydrogène, un méthyle, un halogène, un nitro, un phénylsulfonyle ou un méthoxy,

X représente –COOH, –COCl, $–O–C_pH_{2p}–COOH$ avec p = 1 à 4, $–NH_2$, –OH, $–CH_2NH_2$, $–CH_2OH$ ou 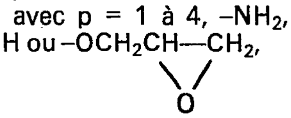

X' représente l'hydrogène ou a la même signification que X, ces symboles ayant des significations telles que l'hydroxyanthraquinone soit exclue,

a représente un nombre de 1 à 100,

b représente un nombre de 0 à 150,

b' représente un nombte de 0,1 à 150,

R représente un radical $–C_mH_{2m}–$ dans lequel m est un nombre de 2 à 12, un radical $–(CH_2CH_2O)_r–CH_2CH_2–$ dans lequel r désigne un nombre de 1 à 40, un radical $–CH(CH_3)CH_2OCH_2CH(CH_3)–$, cyclohexylène,

$–CH_2–C(CH_3)_2–OCOC(CH_3)_2CH_2–$, 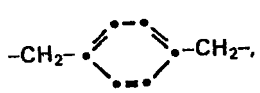

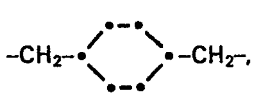 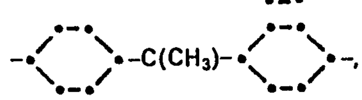

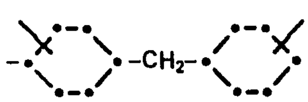 ,

naphthylène ou biphénylylène ou un radical phénylène éventuellement porteur d'un radical méthyle, méthoxy ou nitro,

$R_1$ représente la liaison directe, un radical $-C_mH_{2m}-$ dans lequel m est un nombre de 2 à 12 ou un radical cyclohexylène, cyclohexénylène, phénylène ou endométhylène-cyclohexénylène qui peut porter un radical méthyle,

$R_2$ représente un radical $-C_mH_{2m}-$ dans lequel m désigne un nombre de 2 à 12, un radical phénylène,

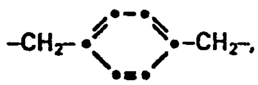 , 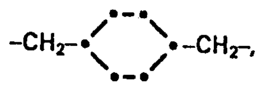 ,

ou un radical répondant à l'une des formules:

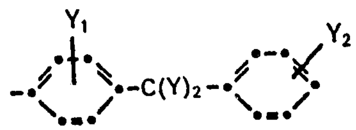 et $-(Y_3O)_o-Y_3-$ ,

Y représente l'hydrogène ou un méthyle,

$Y_1$ et $Y_2$ représentent chacun, indépendamment l'un de l'autre l'hydrogène, le chlore ou le brome,

$Y_3$ représente $-(CH_2)_2-$, $-CH_2CH(CH_3)-$ ou $-(CH_2)_4$,

o représente un nombre de 1 à 50 et

$R_3$ représente:

$R_3$ 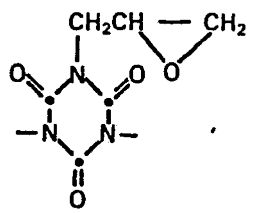 , 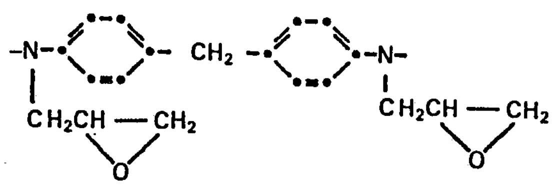

ou le radical de l'éthylène-urée, de la propylène-1,3 urée, de la diméthyl-5,5 hydantoïne, de l'(hydroxy-2 éthyl)- diméthyl-5,5 hydantoïne ou de l'(hydroxy-2 propyl)- diméthyl-5,5 hydantoïne.

2. Mélanges selon la revendication 1 qui contiennent une anthraquinone de formule I, un ou plusieurs composés à radicaux glycidyles terminaux et/ou un composé de formule V, et éventuellement un réticulant et/ou un sel métallique conforme à la définition, et pour lesquels R' et R'' représentent chacun l'hydrogène, X représente un radical $-CH_2NH_2$, $-O-C_pH_{2p}-COOH$ ou $-OH$, X' représente l'hydrogène ou un radical $-O-C_pH_{2p}-COOH$ ou $-OH$ en position 6, b' un nombre de 0,1 à 100 et b un nombre de 1 à 100.

3. Mélanges selon la revendication 1 qui contiennent une anthraquinone de formule I dans laquelle R' et R'' représentent chacun l'hydrogène, X représente un radical $-CH_2NH_2$ ou $-OH$ et X' représente l'hydrogène ou un radical $-OH$ occupant la position 6, un ou plusieurs composés pris dans l'ensemble constitué par les éthers diglycidyliques et/ou triglycidyliques de novolaques phénoliques ou crésoliques, l'isocyanurate de triglycidyle, l'hexahydrophtalate de diglycidyle, la N,N'-diglycidyl diméthyl-5,5 hydantoïne, la N-glycidyl-N'-(glycidyloxy-2 éthyl) diméthyl-5,5 hydantoïne, la N-glycidyl-N'-(glycidyloxy-2 propyl) diméthyl-5,5 hydantoïne et les composés de formules A, B, C et D:

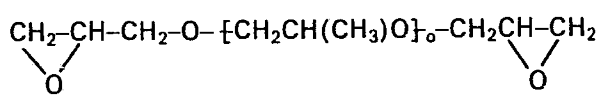 (A),

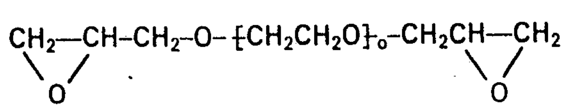 (B),

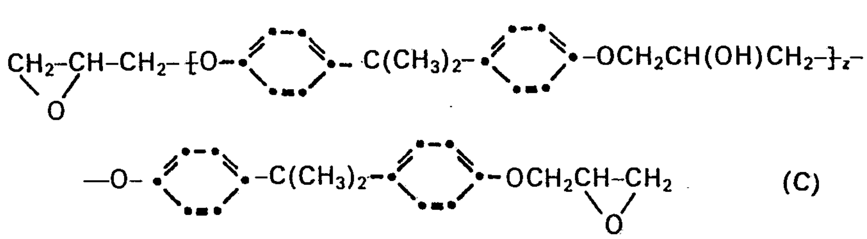 (C)

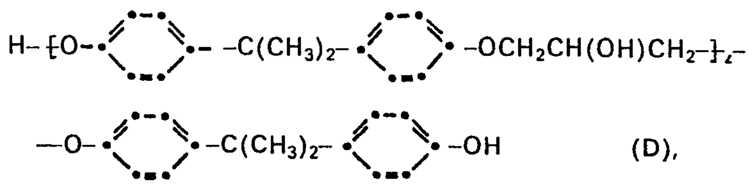

(D),

où désigne un nombre de 1 à 40 et z un nombre de 0,1 à 13, et éventuellement un réticulant et/ou un sel métallique conforme à la définition.

4. Mélange selon la revendication 1 qui contiennent une anthraquinone de formule I dans laquelle R' et R" représentent chacun l'hydrogène, X représente un radical -OH et X' représente l'hydrogène ou un radical -OH occupant la position 6 un composé répondant à l'une des formules A et B:

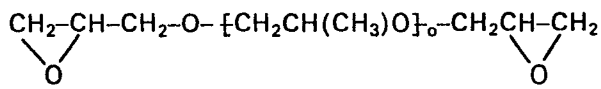

(A)

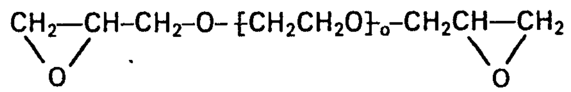

(B)

en mélange avec un composé de formule C:

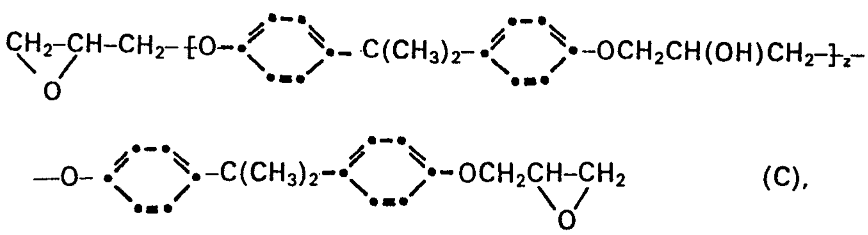

(C),

(dans les formules o désigne un nombre de 2 à 20 et z un nombre de 2 à 11), et éventuellement un réticulant et/ou un sel métallique conforme à la définition.

5. Mélanges selon la revendication 1 qui contiennent une anthraquinone de formule I dans laquelle R' et R" représentent chacun l'hydrogène, X représente un radical -OH et X' représente l'hydrogène ou un radical -OH occupant la position 6, de la N,N'-diglycidyl diméthyl-5,5 hydantoïne, de la N-glycidyl-N'-(glycidyloxy-2 éthyl) diméthyl-5,5 hydantoïne et/ou de la N-glycidyl-N'-(glycidyloxy-2 propyl) diméthyl-5,5 hydantoïne en mélange avec un composé de formule C:

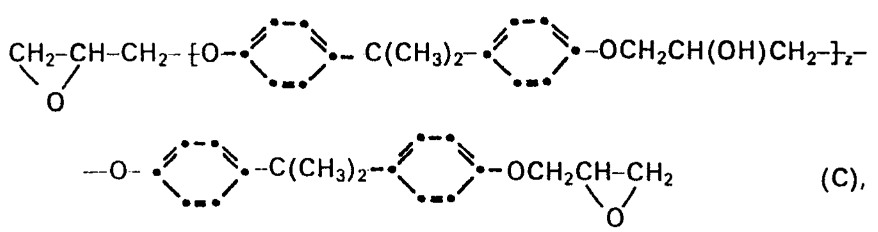

(C),

(z désigne un nombre de 2 à 11 et o un nombre de 2 à 20), et éventuellement un réticulant et/ou un sel métallique conforme à la définition).

6. Mélanges selon la revendication 1 qui contiennent un sel de fer, de cobalt, de nickel ou de cuivre.

7. Mélange selon la revendication 1 qui contiennent de l'acétate de cuivre(II) ou un mélange d'acétate de cuivre(II) et de bromure de cuivre(II).

8. Mélanges selon la revendication 1 qui contiennent, comme réticulant, de l'anhydride hexahydrophtalique ou du bis-phénol A.

9. Produits réactionnels photosensibles, éventuellement réticulés, que l'on peut obtenir en faisant réagir, éventuellement en présence d'un réticulant, un composé de formule I avec un ou plusieurs composés pris dans l'ensemble constitué par les éthers diglycidyliques et polyglycidyliques de novolaques phénoliques ou crésoliques et les composés de formules II à V qui portent des radicaux terminaux fonctionnels appropriés, et éventuellement des composés de formules VI, VII et/ou VIII selon la revendication 1, la proportion des composés de formules VI à VIII étant d'au plus 80% en moles par rapport à tous les composés indiqués sous 2) à la revendication 1, puis, le cas échéant, en complexant les produits réactionnels obtenus, au moins partiellement, par un sel d'un métal du groupe Ib ou VIII de la classification périodique.

10. Produits réactionnels photosensibles selon la revendication 9, caractérisés en ce que les produits réactionnels obtenus ont été complexés, au moins partiellement, par un sel de fer, de cobalt, de nickel ou de cuivre.

11. Produits réactionnels photosensibles selon la revendication 9, caractérisés en ce que les produits réactionnels obtenus ont été complexés, au moins partiellement, par de l'acétate de cuivre(II) ou par un mélange d'acétate de cuivre(II) et de bromure de cuivre(II).

12. Produits réactionnels photosensibles selon la revendication 9, caractérisés en ce qu'ils ont été préparés en présence d'un réticulant qui est l'anhydride hexahydrophtalique ou le bis-phénol A.

13. Produits réactionnels photosensibles selon la revendication 9, caractérisés en ce qu'ils sont des polymères contenant des motifs répondant à l'une des formules IX, X et XI:

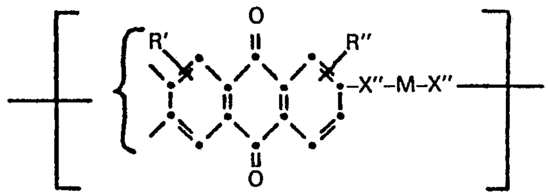
(IX),

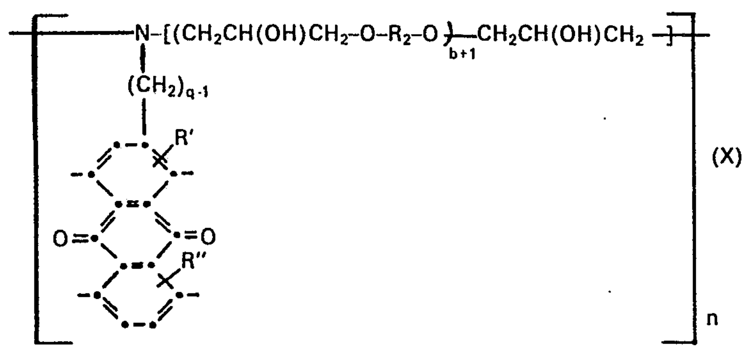
(X)

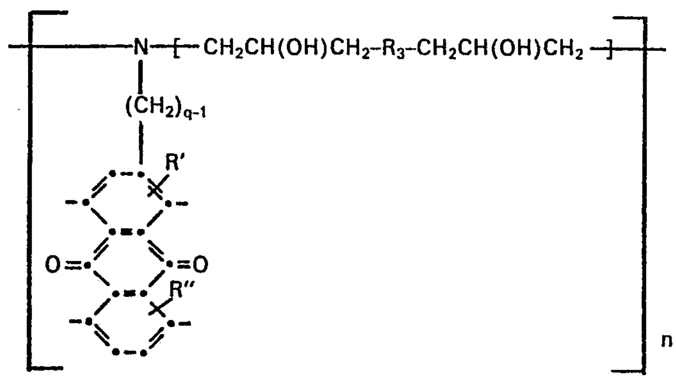
(XI)

ou des composés répondant à l'une des formules XII et XIII:

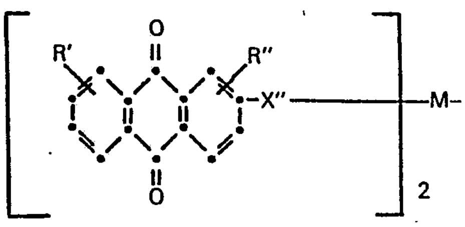
(XII)

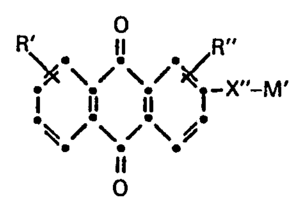
(XIII),

éventuellement complexés, au moins partiellement, par des ions métalliques d'un métal du groupe Ib ou VIII de la classification périodique, formules dans lesquelles:

R' et R'' représentent chacun indépendamment l'un de l'autre l'hydrogène, un méthyle, un halogène, un nitro, un phénylsulfonyle ou un méthoxy, X'' représente un radical $-CO-$, $-O-C_pH_{2p}-CO-$ (p étant un nombte de 1 à 4), $-NH-$, $-N'-$, $-O-$, $-CH_2NH-$, $-CH_2N'-$, $CH_2O-$ ou $-OCH_2$

$CH(OH)CH_2-$, les M représentent: dans le cas où X'' représente un radical $-CO-$ ou $-O-C_pH_{2p}-CO-$, chacun indépendamment les uns des autres un radical d'éther di- ou polyglycidylique d'une novolaque phénolique ou crésolique, relié par $-O-$, ou un radical de formule IIa, IIIa, IVa ou Va:

$-OCH_2CH(OH)CH_2-O-CO-R_1-CO-O-CH_2CH(OH)CH_2O-$ (IIa),

$-OCH_2CH(OH)CH_2-[O-R_2-O-CH_2CH(OH)CH_2]_b-O-R_2-O-CH_2CH(OH)CH_2O-$ (IIIa),

$-OCH_2CH(OH)CH_2-R_3-CH_2CH(OH)CH_2O-$ (IVa)

$-O-R_2-O-[CH_2CH(OH)CH_2-O-R_2-O]_{b'}-$ (Va)

et éventuellement, en partie, un radical de formule VIa ou VIIIa:

$-O-R-O-[CO-R_1-CO-O-RO]_a-$ (VIa)

$-O-[Y_3-O]_o-Y_3-O-$ (VIIIa),

dans le cas où X'' représente un radical $-NH-$, $-O-$, $-CH_2NH-$ ou $-CH_2O-$, chacun indépendamment les uns des autres un radical d'éther di- ou polyglycidylique d'une novolaque phénolique ou crésolique, ou un radical de formule IIb, IIIb ou IVb:

$-CH_2CH(OH)CH_2-O-CO-R_1-CO-O-CH_2CH(OH)CH_2-$ (IIb),

$-CH_2CH(OH)CH_2-[O-R_2-O-CH_2CH(OH)CH_2]_b-$ (IIIb)

$-CH_2CH(OH)CH_2-R_3-CH_2CH(OH)CH_2-$ (IVb)

et éventuellement, en partie, un radical de formule VIIa:

$-OC-R_1-CO-[O-R-O-CO-R_1-CO]_a-$ (VIIa),

dans le cas où X'' représente un radical $-OCH_2CH(OH)CH_2-$, chacun indépendamment les uns des autres un radical de formule Va, ou éventuellement, en partie, un radical de formule VIa, VIIa et/ou VIIIa, et dans le cas où X'' représente un radical $-\overset{|}{N}'-$ ou $-CH_2\overset{|}{N}'-$, chacun indépendamment les uns des autres un radical d'éther di- ou polyglycidylique d'une novolaque phénolique ou crésolique, ou un radical de formule IIb, IIIb ou IVb, les M' représentent: dans le cas où X'' représente un radical $-CO-$ ou $-O-C_pH_{2p}-CO-$, chacun indépendamment les uns des autres un radical de formules IIIc ou Vb:

$-OCH_2CH(OH)CH_2-[O-R_2-O-CH_2CH(OH)CH_2]_{b''}-O-R_2O-CH_2CH—CH_2$ (IIIc)
O (époxyde entre CH et $CH_2$)

$-O-R_2-O-[CH_2CH(OH)CH_2-O-R_2-O]_{b''}-H$ (Vb)

ou éventuellement, en partie, un radical de formule VIb ou VIIIb:

$-O-R-O-[CO-R_1-CO-O-R-O]_{a'}-H$ (VIb)

$-O-[Y_3-O]_{o'}-Y_3-OH$ (VIIIb),

dans le cas où X'' représente un radical $-NH-$, $-O-$, $-CH_2NH-$ ou $-CH_2O-$, chacun indépendamment les uns des autres un radical de formule IIId:

$-CH_2CH(OH)CH_2-[O-R_2-O-CH_2CH(OH)CH_2]_{b''}-O-R_2-O-CH_2CH—CH_2$ (IIId)
O (époxyde entre CH et $CH_2$)

ou éventuellement, en partie, un radical de formule VIIb:

$-OC-R_1-CO-[O-R-O-CO-R_1-CO]_{a'}-OH$ (VIIb),

dans le cas où X'' représente un radical $-OCH_2CH(OH)CH_2-$, chacun indépendamment les uns des autres un radical de formule Vb ou éventuellement, en partie, un radical de formule VIb, VIIb ou VIIIb, et dans le cas où X'' représente un radical $-\overset{|}{N}'-$ ou $-CH_2\overset{|}{N}'-$, chacun indépendamment les uns des autres un radical de formule IIId, a représente un nombre de 1 à 100, b représente un nombre de 0 à 150, b' représente un nombre de 0,1 à 150, q représente un nombre égal à 1 ou à 2,

b″ représente un nombre de 5 à 150, a′ représente un nombre de 5 à 100, o′ représente un nombre de 5 à 50, R représente un radical $-C_mH_{2m}-$ dans lequel m est un nombre de 2 à 12, un radical $-(CH_2CH_2O-)_r-CH_2CH_2-$ dans lequel r désigne un nombre de 1 à 40, un radical $-CH(CH_3)CH_2OCH_2CH(CH_3)-$, cyclohexylène,

$-CH_2-C(CH_3)_2-OCOC(CH_3)_2CH_2-$, 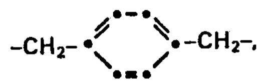

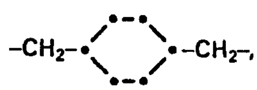 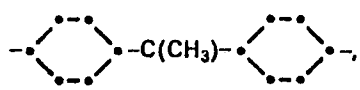

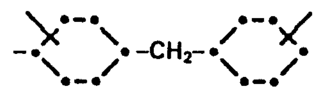 .

naphtylène ou biphénylylène ou un radical phénylène éventuellement porteur d'un radical méthyle, méthoxy ou nitro,

$R_1$ représente la liaison directe, un radical $-C_mH_{2m}-$ dans lequel m est un nombre de 2 à 12 ou un radical cyclohexylène, cyclohexénylène, phénylène ou endométhylène-cyclohexénylène qui peut porter un radical méthyle,

$R_2$ représente un radical $-C_mH_{2m}-$ dans lequel m désigne un nombre de 2 à 12, un radical phénylène,

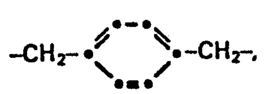 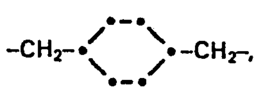

ou un radical répondant à l'une des formules:

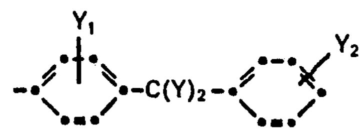 oder $-(Y_3O)_o-Y_3-$ ,

Y représente l'hydrogène ou un méthyle,

$Y_1$ et $Y_2$ représentent chacun, indépendamment l'un de l'autre, l'hydrogène, le chlore ou le brome,

$Y_3$ représente $-(CH_2)_2-$, $-CH_2CH(CH_3)-$ ou $-(CH_2)_4$,

o représente un nombre de 1 à 50 et

$R_3$ représente:

$R_3$ 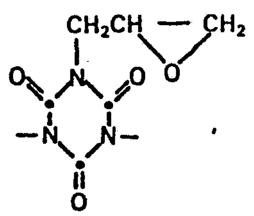 , 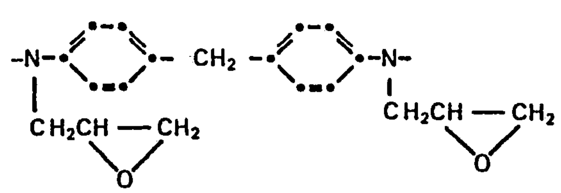

ou le radical de l'éthylène-urée, de la propylène-1,3 urée, de la diméthyl-5,5 hydantoïne, de l'(hydroxy-2 éthyl)- diméthyl-5,5 hydantoïne ou de l'(hydroxy-2 propyl)- diméthyl-5,5 hydantoïne, le symbole X″, dans les formules XII et XIII, n'étant pas –O–.

14. Produits réactionnels photosensibles selon la revendication 13, qui sont complexés, au moins partiellement, par des ions de fer, de cobalt, de nickel et de cuivre.

15. Produits réactionnels photosensibles selon la revendication XIII, qui sont complexés, au moins partiellement, par des ions $Cu^{++}$.

16. Anthraquinones répondant à la formule I′:

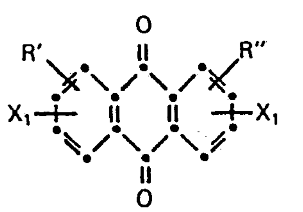 (I′)

dans laquelle R′ et R″ représentent chacun, indépendamment l'un de l'autre, l'hydrogène, un méthyle, un halogène, un nitro, un phénylsulfonyle ou un méthoxy, l'un des symboles $X_1$ représente l'hydrogène ou un radical $-OCH_2CH—CH_2$ (époxy, O) et l'autre représente un radical $-OCH_2CH—CH_2$ (O), ou R′ et R″ représentent chacun un atome d'hydrogène, l'un des $X_1$ représente un atome d'hydrogène et l'autre représente un radical aminométhyle occupant la position 2.

17. Anthraquinones de formule I′ selon la revendication 16, dans lesquelles il y a un radical $-OCH_2CH—CH_2$ (O) à la position 2 ou a chacune des positions 2 et 6.

18. Application de mélanges selon la revendication 1 qui ne contiennent pas de sel métallique conforme à la définition, ou de polymères ou de composés selon la revendication 13 qui ne sont pas complexés avec des ions d'un métal du groupe Ib ou VIII de la classification périodique, pour la fabrication de revêtements ou de dessins électroconducteurs par irradiation et dépôt non électrolytique de métaux.

**Claims**

1. A photosensitive composition which is capable of undergoing condensation or addition reactions and may or may not be crosslinkable, containing

1) an anthraquinone of the formula I

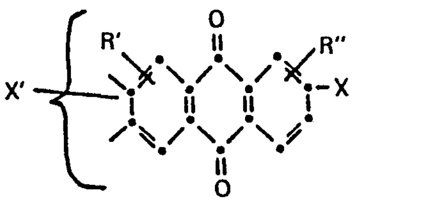 (I),

2) one or more compounds selected from di- to poly-glycidyl ethers of phenol and cresol novolaks and compounds of the formulae II to VIII

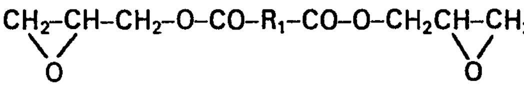 (II),

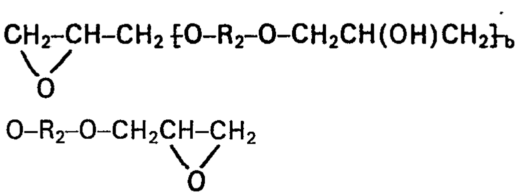 III),

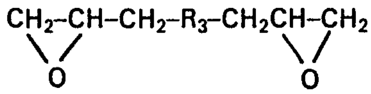 (IV),

$HO-R_2-O[CH_2CH(OH)CH_2-O-R_2-O]_{b'}-H$ (V),

$HO-R-O[CO-R_1-CO-O-R-O]_aH$ (VI),

$HOOC-R_1-CO[O-R-O-CO-R_1-CO]_aOH$ (VII)

and

$HO[Y_3-O]_o-Y_3-OH$ (VIII),

the amount of compounds of the formulae VI, VII and/or VIII being not more than 80 mol%, based on all the reactants mentioned under 2),

3) if appropriate, a crosslinking agent and

4) if appropriate, a salt of a metal of group Ib or VIII of the periodic table,

in which each of R' and R'' independently of the other is hydrogen, methyl, halogen or a nitro, phenylsulfonyl or methoxy group, X is $-COOH$, $-COCl$, $-O-C_pH_{2p}-COOH$, where p = 1 to 4, $-NH_2$, $-OH$, $-CH_2NH_2$, $-CH_2OH$ or 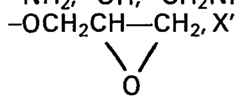, X' is hydrogen or has the same meaning as X with the exception of hydroxyanthraquinone, a is a number from 1 to 100, b is a number from 0 to 150, b' is a number from 0.1 to 150, R is $-C_mH_{2m}-$, where m = 2–12, $(CH_2CH_2O)_r-CH_2CH_2-$, where r = 1–40, $-CH(CH_3)CH_2OCH_2CH(CH_3)-$, cyclohexylene,

$-CH_2-C(CH_3)_2-OCOC(CH_3)_2CH_2-$, 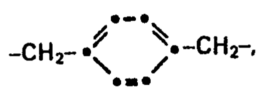

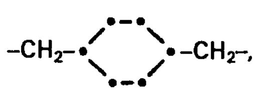 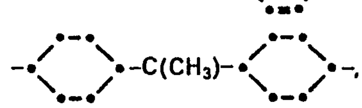

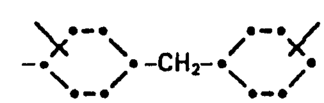 ,

naphthylene, biphenylene, or phenylene which is unsubstituted or substituted by a methyl, methoxy or nitro group, $R_1$ is a direct bond, $-C_mH_{2m}-$, where m = 2–12, or cyclohexylene, cyclohexenylene, phenylene or endomethylenecyclohexenylene, each of which may be substituted by a methyl group, $R_2$ is $-C_mH_{2m}-$, where m = 2–12, phenylene,

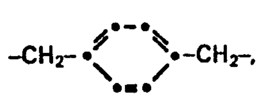 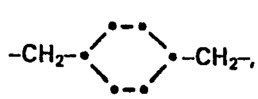

or a group of the formula

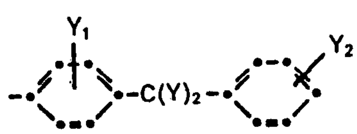 or $-(Y_3O)_o-Y_3-$ .

Y is hydrogen or methyl, each of $Y_1$ and $Y_2$ independently of the other is hydrogen, chlorine or bromine, $Y_3$ is $-(CH_2)_2-$, $-CH_2CH(CH_3)-$ or $-(CH_2)_4-$, o is a number from 1 to 50 and $R_3$ is

$R_3$ 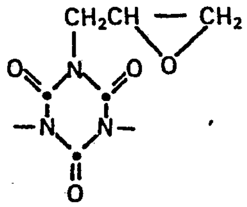 , 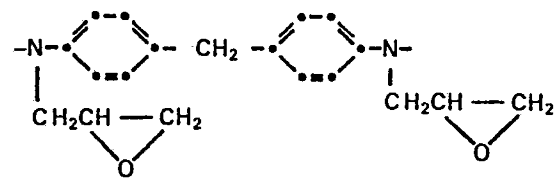

or the radical of ethyleneurea, 1,3-propyleneurea, 5,5-dimethylhydantoin, 2-hydroxyethyl-5,5-dimethylhydantoin or 2-hydroxypropyl-5,5-dimethylhydantoin.

2. A composition according to claim 1, containing an anthraquinone of the formula I, one or more compounds with glycidyl end groups and/or a compound of the formula V, and, if appropriate, a crosslinking agent and/or a metal salt of the type defined, in which R' and R" are hydrogen, X is $-CH_2NH_2$, $-O-C_pH_{2p}-COOH$ or $-OH$, X' is hydrogen or $-O-C_pH_{2p}-COOH$ or $-OH$ bonded in the 6-position, b' is a number from 0.1 to 100 and b is a number from 1 to 100.

3. A composition according to claim 1, containing an anthraquinone of the formula I in which R' and R" are each hydrogen, X is $-CH_2NH_2$ or $-OH$ and X' is hydrogen or $-OH$ which is bonded in the 6-position, one or more compounds selected from di- and/or tri-glycidyl ethers of phenol or cresol novolaks, triglycidyl isocyanurate, diglycidyl hexahydrophthalate, N,N'-diglycidyl-5,5-dimethylhydantoin, N-glycidyl-N'-2-hydroxyethylglycidyl- and/or N-glycidyl-N'-2-hydroxypropylglycidyl-5,5-dimethylhydantoin and cumpounds of the formulae (A7, B), C) and D)

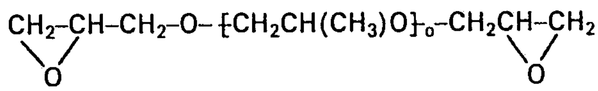

(A),

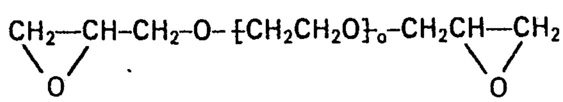

(B),

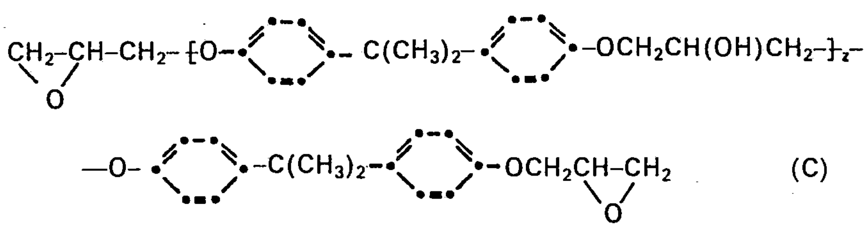

(C)

and

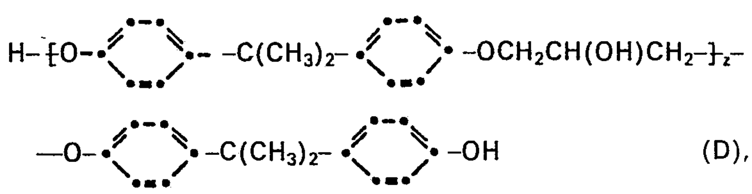

(D),

and, if appropriate, a crosslinking agent and/or a metal salt of the type defined, in which o is 2–40 and z is 0.1–13.

4. A composition according to claim 1, containing an anthraquinone of the formula I in which R' and R" are hydrogen, X is $-OH$ ans X' is hydrogen or $-OH$ which is bonded in the 6-position, a compound of the formula A) or B)

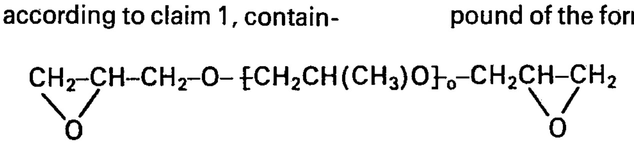

(A) or

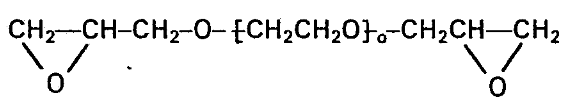

(B)

in admixture with a compound of the formula C)

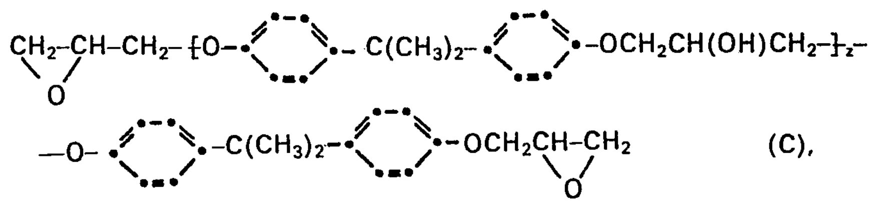

(C),

and, if appropriate, a crosslinking agent and/or a metal salt of the type defined, in which o is 2–20 and z is 2–11.

5. A composition according to claim 1, containing an anthraquinone of the formula I in which R' and R" are hydrogen, X is –OH and X' is hydrogen or –OH which is bonded in the 6-position, N,N'-diglycidyl-5,5-dimethylhydantoin, N-glycidyl-N'-2-hydroxyethyl and/or N-glycidyl-N'-2-hydroxypropylglycidyl- 5,5-dimethylhydantoin, in admixture with a compound of the formula C)

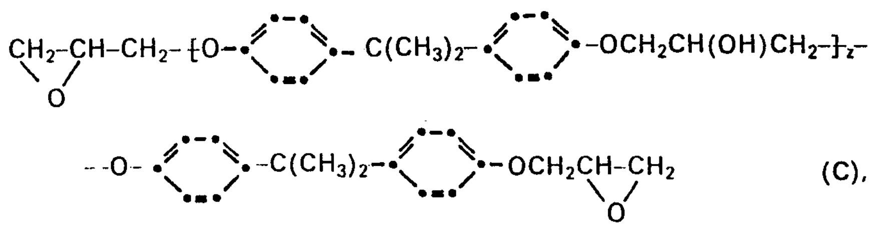

(C),

and, if appropriate, a crosslinking agent and/or a metal salt of the type defined, in which o is 2–20 and z is 2–11.

6. A composition according to claim 1, containing an iron, cobalt, nickel or copper salt.

7. A composition according to claim 1, containing copper-II acetate or a mixture of copper-II acetate and copper-II bromide.

8. A composition according to claim 1, containing hexahydrophthalic anhydride or bisphenol A as the crosslinking agent.

9. A photosensitive reaction product, which may or may not be crosslinked, which can be obtained by reacting a compound of the formula I with one or more compounds selected from di- to poly-glycidyl ethers of phenol or cresol novolaks and compounds of the formulae II to V with suitable functional end groups and, if appropriate, compounds of the formulae VI, VII and/or VIII according to claim 1, in the presence or absence of a crosslinking agent, the proportion of compounds of the formulae VI to VIII being not more than 80 mol%, based on all the compounds mentioned under 2) in claim 1, and then, if appropriate, at least partly complexing the resultant reaction products with a salt of a metal of group Ib or VIII of the periodic table.

10. A photosensitive reaction product according to claim 9, which is at least partly complexed with an iron, cobalt, nickel or copper salt.

11. A photosensitive reaction product according to claim 9, which is at least partly complexed with copper-II acetate or a mixture of cooper-II acetate and copper-II bromide.

12. A photosensitive reaction product according to claim 9, for which hexahydrophthalic anhydride or bisphenol A is used as the crosslinking agent.

13. A photosensitive reaction product according to claim 9, which is a polymer which contains recurring structural elements of the formula IX, X or XI

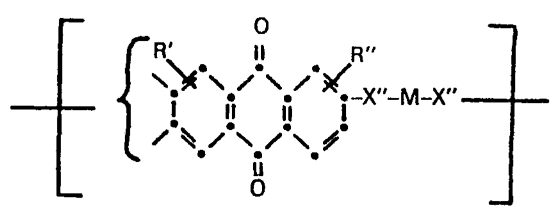

(IX),

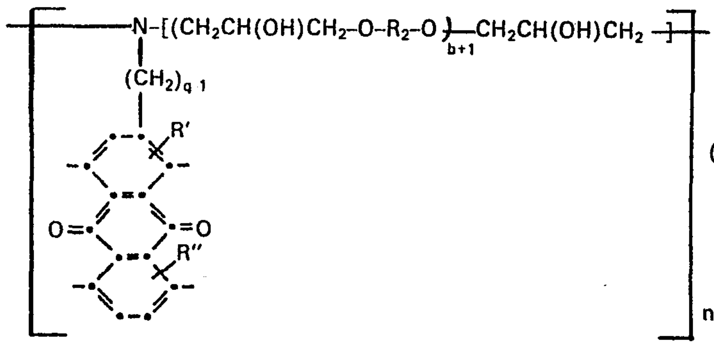

(X)

or

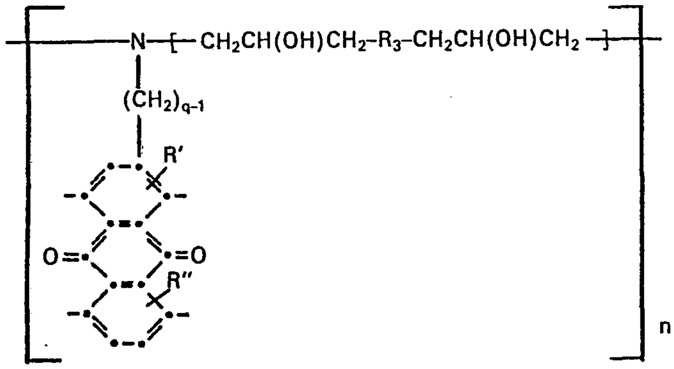

(XI)

or is a compound of the formula XII or XIII

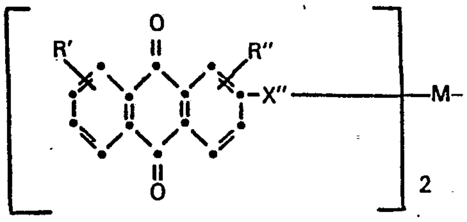

or

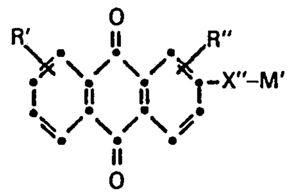

(XII) (XIII),

which can be at least partly complexed with metal ions of a metal of group Ib or VIII of the periodic table, in which each of R′ and R″ independently of the other is hydrogen, methyl, halogen or a nitro, phenylsulfonyl or methoxy group, X″ is $-CO-$, $-O-C_pH_{2p}-CO$, where p = 1 to 4, $-NH-$, $-\overset{|}{N}-$, $-O-$, $-CH_2NH-$, $-CH_2\overset{|}{N}-$, $-CH_2O-$ or $-OCH_2-CH(OH)CH_2-$ and, if X″ is $-CO-$ or $-O-C_pH_{2p}-CO-$, the radicals M are identical or different radicals, bonded via $-O-$, of di- to poly-glycidyl ethers of phenol or cresol novolaks or groupings of the formula IIa, IIIa, IVa or Va

$$-OCH_2CH(OH)CH_2-O-CO-R_1-CO-O-CH_2CH(OH)CH_2O- \quad \text{(IIa)},$$

$$-OCH_2CH(OH)CH_2-[O-R_2-O-CH_2CH(OH)CH_2]_b-O-R_2-O-CH_2CH(OH)CH_2O- \quad \text{(IIIa)},$$

$$-OCH_2CH(OH)CH_2-R_3-CH_2CH(OH)CH_2O- \quad \text{(IVa)}$$

or

$$-O-R_2-O-[CH_2CH(OH)CH_2-O-R_2-O]_{b'}- \quad \text{(Va)}$$

and, if appropriate, in some cases a grouping of the formula VIa or VIIIa $-O-R-O-[CO-R_1-CO-O-R-O]_a-$ (VIa) or $-O-[Y_3-O]_o-Y_3-O-$ (VIIIa) or, if X″ is $-NH-$, $-O-$, $-CH_2NH-$ or $-CH_2O-$, the radicals M are identical or different radicals of di- to poly-glycidyl ethers of phenol or cresol novolaks or groupings of the formula IIb, IIIb or IVb

$$-CH_2CH(OH)CH_2-O-CO-R_1-CO-O-CH_2CH(OH)CH_2- \quad \text{(IIb)},$$

$$-CH_2CH(OH)CH_2-[O-R_2-O-CH_2CH(OH)CH_2]_b- \quad \text{(IIIb)} \text{ or}$$

$$-CH_2CH(OH)CH_2-R_3-CH_2CH(OH)CH_2- \quad \text{(IVb)}$$

and, if appropriate, in some cases a grouping of the formula VIIa

$$-OC-R_1-CO-[O-R-O-CO-R_1-CO]_a- \quad \text{(VIIa)},$$

or, if X″ is $-OCH_2CH(OH)CH_2-$, the radicals M are identical or different groupings of the formula Va and, if appropriate, in some cases groupings of the formulae VIa, VIIa and/or VIIIa, or if X″ is $-\overset{|}{N}-$

or $-CH_2N'-$, the radicals M are identical or different radicals of di- to poly-glycidyl ethers of phenol or cresol novolaks or groupings of the formula IIb, IIIb or IVb, and, if X" is –CO– or $-O-C_pH_{2p}-CO-$, the radicals M' are identical or different groupings of the formula IIIc or Vb

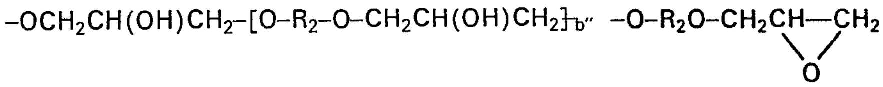

(IIIc)

or

$-O-R_2-O-[CH_2CH(OH)CH_2-O-R_2-O]_{b''}-H$ (Vb)

and, if appropriate, in some cases a grouping of the formula VIb or VIIIb

$-O-R-O-[CO-R_1-CO-O-R-O]_{a'}-H$ (VIb)

or

$-O-[Y_3-O]_{o'}-Y_3-OH$ (VIIIb),

or, if X" is –NH–, –O–, $-CH_2NH-$ or $-CH_2O-$, the radicals M' are identical or different groupings of the formula IIId

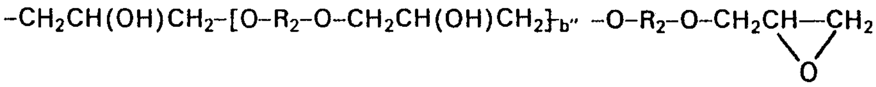

(IIId)

and, if appropriate, in some cases a grouping of the formula VIIb

$-OC-R_1-CO-[O-R-O-CO-R_1-CO]_{a'}-OH$ (VIIb),

or, if X" is $-OCH_2CH(OH)CH_2-$, the radicals M' are identical or different groupings of the formula Vb and, if appropriate, in some cases a grouping of the formulae VIb, VIIb and/or VIIIb, or, if X" is –N'– or $-CH_2N'-$, the radicals M' are identical or different groupings of the formula IIId, and a is a number from 1 to 100, b is a number from 0 to 150, b' is a number from 0.1 to 150, q is the number 1 or 2, b" is a number from 5 to 150, a' is a number from 5 to 100, o' is a number from 5 to 50, R is $-C_mH_{2m}$, where m = 2–12, $-(CH_2CH_2O)_r-CH_2CH_2-$, where r = 1–40, $-CH(CH_3)CH_2OCH_2CH(CH_3)-$, cyclohexylene,

$-CH_2-C(CH_3)_2-OCOC(CH_3)_2CH_2-$, 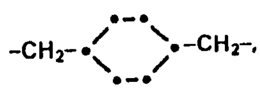

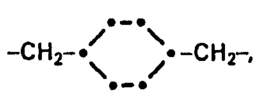 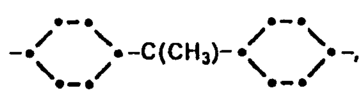

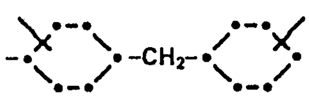 ,

naphthylene, biphenylene, or phenylene which is unsubstituted or substituted by a methyl, methoxy or nitro group, $R_1$ is a direct bond, $-C_mH_{2m}-$, where m = 2–12, or cyclohexylene, cyclohexenylene, phenylene or endomethylenecyclohexenylene, each of which may be substituted by a methyl group, $R_2$ is $-C_mH_{2m}-$, where m = 2–12, phenylene,

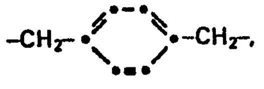 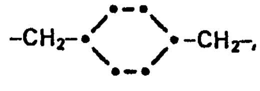

or a group of the formula

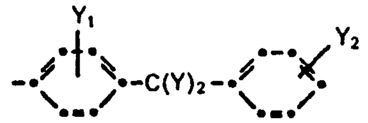 or $-(Y_3O)_o-Y_3-$ ,

Y is hydrogen or methyl, each of $Y_1$ and $Y_2$ independently of the other is hydrogen, chlorine or bromine, $Y_3$ is $-(CH_2)_2-$, $-CH_2CH(CH_3)-$ or $-(CH_2)_4-$, o is a number from 1 to 50 and $R_3$ is

$R_3$ 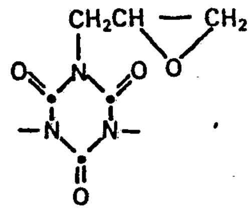 , 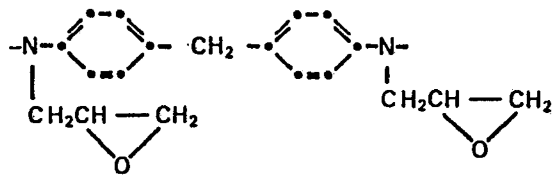

or the radical of ethyleneurea, 1,3-propyleneurea, 5,5-dimethylhydantoin, 2-hydroxyethyl-5,5-dimethylhydantoin or 2-hydroxypropyl-5,5-dimethylhydantoin, with the proviso that X'' in formulae XII and XIII is not oxygen.

14. A photosensitive reaction product according ti claim 13, which is at least partly complexed with iron, cobalt, nickel or copper ions.

15. A photosensitive reaction product according to claim 13, which is at least partly complexed with $Cu^{++}$ ions.

16. An anthraquinone of the formula I'

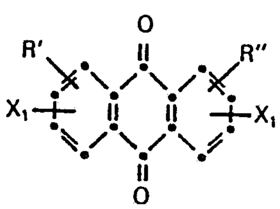 (I')

in which each of R' and R'' independently of the other is hydrogen, methyl, halogen or a nitro, phenylsulfonyl or methoxy group and one of the radicals $X_1$ is hydrogen or $-OCH_2CH—CH_2$ and the other is $-OCH_2CH—CH_2$, or R' and R'' are a hydrogen atom and one of the radicals $X_1$ is a hydrogen atom and the other is aminomethyl bonded in the 2-position.

17. An anthraquinone of the formula I' according to claim 16, in which the $-OCH_2CH—CH_2$ groups are bonded in the 2- or in the 2- and 6-position.

18. The use of a composition according to claim 1 containing no metal salt of the type defined, or of a polymer or compound according to claim 13 which is not complexed with metal ions of a metal of group Ib or VIII of the periodic table, for the production of electrically conductive coatings or patterns by exposure to light and electroless deposition of metals.